# EUROPEAN PATENT SPECIFICATION

(11) **EP 3 810 159 B1**
(45) Date of publication and mention of the grant of the patent: **16.10.2024**
(21) Application number: 19795975.2
(22) Date of filing: 03.05.2019
(51) Int. Cl.: A61K 35/33, A61K 38/17, A61P 35/00

(54) **FIBROBLAST DELIVERY OF TUMOR INHIBITORY AGENTS**
FIBROBLASTENABGABE VON TUMORHEMMENDEN MITTELN
LIBÉRATION DE FIBROBLASTES D'AGENTS INHIBITEURS DE TUMEUR

(30) Priority: 04.05.2018 US 201862666786 P
(43) Date of publication of application: 28.04.2021
(60) Divisional application: 24199539.8
(73) Proprietor: FibroBiologics, Inc., Houston, Texas 77289 (US)
(72) Inventor: O'HEERON, Pete, Houston, TX 77059 (US); ICHIM, Thomas, Houston, Texas 77058 (US)
(74) Representative: J A Kemp LLP
(86) International application number: PCT/US2019/030585
(87) International publication number: WO 2019/213510

(56) References cited:
- WO-A1-2015/035235
- WO-A1-2018/064323
- US-A- 6 096 303
- TAHARA H ET AL: "Fibroblasts genetically engineered to secrete Interleukin 12 can suppress tumor growth and induce antitumor immunity to a murine melanoma in vivo", CANCER RESEARCH, AMERICAN ASSOCIATION FOR CANCER RESEARCH, US, vol. 54, 1 January 1994 (1994-01-01), pages 182 - 189, XP002128300, ISSN: 0008-5472
- KALIMUTHU SENTHILKUMAR ET AL: "In Vivo Tracking of Chemokine Receptor CXCR4-Engineered Mesenchymal Stem Cell Migration by Optical Molecular Imaging", STEM CELLS INTERNATIONAL, vol. 2017, 1 January 2017 (2017-01-01), US, pages 1 - 10, XP055870244, ISSN: 1687-966X, Retrieved from the Internet <URL:https://downloads.hindawi.com/journals/sci/2017/8085637.pdf> DOI: 10.1155/2017/8085637
- GUO Z S ET AL: "Oncolytic virotherapy: Molecular targets in tumor-selective replication and carrier cell-mediated delivery of oncolytic viruses", BBA - REVIEWS ON CANCER, ELSEVIER SCIENCE BV, AMSTERDAM, NL, vol. 1785, no. 2, 1 April 2008 (2008-04-01), pages 217 - 231, XP022615368, ISSN: 0304-419X, [retrieved on 20080215], DOI: 10.1016/J.BBCAN.2008.02.001
- D. ZAMARIN ET AL: "Localized Oncolytic Virotherapy Overcomes Systemic Tumor Resistance to Immune Checkpoint Blockade Immunotherapy", SCIENCE TRANSLATIONAL MEDICINE, vol. 6, no. 226, 5 March 2014 (2014-03-05), pages 226ra32 - 226ra32, XP055296235, ISSN: 1946-6234, DOI: 10.1126/scitranslmed.3008095
- QU, Y ET AL.: "Enhanced migration and CXCR4 over-expression in fibroblasts with telomerase reconstitution", MOLECULAR AND CELLULAR BIOCHEMISTRY, vol. 313, no. 1-2, 25 March 2008 (2008-03-25), pages 45 - 52, XP019611452, ISSN: 1573-4919
- HOSOKAWA, Y ET AL.: "CXCL12 and CXCR4 expression by human gingival fibroblasts in periodontal disease", CLINICAL AND EXPERIMENTAL IMMUNOLOGY, vol. 141, no. 3, September 2005 (2005-09-01), pages 467 - 474, XP055649358, ISSN: 0009-9104, DOI: 10.1111/j.1365-2249.2005.02852.x

## Description

### TECHNICAL FIELD

Described herein is an *in vitro* method of preparing fibroblasts as anti-cancer delivery compositions, and fibroblasts prepared by the method for use in a method of treating cancer.

### BACKGROUND

It is known that growth and metastasis of various types of cancers is associated with a number of characteristic cellular and molecular changes in the surrounding stroma cells. One highly consistent feature of the reactive stroma of numerous types of epithelial cell cancer is the induction of the fibroblast activating protein alpha (hereafter referred to as FAP-alpha or FAP), a cell surface molecule of the reactive stromal fibroblast that was originally identified with the monoclonal antibody F 19. Because the FAP is selectively expressed in stroma of a number of epithelial cell carcinomas, irrespective of the site and histological type of the carcinoma, it was desirable to develop a treatment aspect for a FAP-alpha target molecule in order to allow imaging techniques, the diagnosis and treatment of cancer, such as epithelial cell cancer, and many other syndromes. The findings that cancer-associated fibroblasts possess various markers of activation has indicated aspects wherein there is a specific affinity of fibroblasts toward tumor tissues.

Tahara H et al (Cancer Research, American Association For Cancer Research, US, vol. 54, 1 January 1994, pages 182-189) relates to the use of fibroblasts genetically engineered to secrete Interleukin 12 for suppressing tumor growth and inducing antitumor immunity in a murine melanoma model in vivo. Kalimuthu Senthilkumar et al (Stem Cells International, vol. 2017, 1 January 2017, pages 1 -10) relates to a method of *in vivo* tracking of chemokine receptor CXCR4-engineered mesenchymal stem cell migration by optical molecular imaging. WO 2015/035235 relates to compositions and methods for treating a subject with cancer and/or increasing migration of a mesenchymal stromal cells (MSCs) stimulated with a recombinant autocrine motility factor (rAMF) to a tumor or a tumor cell. Guo Z S et al (BBA - Reviews On Cancer, Elsevier Science Bv, Amsterdam, NL, vol. 1785, no. 2, 1 April 2008, pages 217-231) relates to molecular targets in tumor-selective replication and carrier cell-mediated delivery of oncolytic viruses. D. Zamarin et al (Science Translational Medicine, vol. 6, no. 226, 5 March 2014, pages 226ra32-226ra32) relates to the use of a localized oncolytic virotherapy to overcome systemic tumor resistance to immune checkpoint blockade immunotherapy.

There is a long-felt need in the art for effective cancer treatment. The disclosure provides the unexpected use of fibroblasts and manipulated fibroblasts as a means of treating cancer.

### SUMMARY OF THE INVENTION

The invention provides an *in vitro* method of preparing fibroblasts as anti-cancer delivery compositions, comprising the steps of: (a) modifying fibroblasts to enhance activity of homing to cancer cells or tissue comprising cancer cells; and (b) manipulating fibroblasts to comprise one or more tumor inhibitory agents, wherein the fibroblasts of step (a), step (b), or both are exposed to hypoxia under suitable conditions and wherein the tumor inhibitory agent comprises a vector that expresses interleukin-12 (IL-12).

The invention also provides fibroblasts prepared by the method of the invention for use in a method of treating cancer, wherein the method comprises administering an effective amount of the modified fibroblasts to an individual.

The invention is defined in the independent claims. Further aspects and preferred embodiments are defined in the dependent claims. Any aspects, embodiments and examples of the present disclosure which do not fall under the scope of the appended claims do not form part of the invention and are merely provided for illustrative purposes.

References to the methods of treatment by therapy or surgery or *in vivo* diagnosis methods in the description are to be interpreted as references to compounds, pharmaceutical compositions and medicaments of the present invention for use in those methods.

The present disclosure is directed to methods and compositions for the treatment of cancer of any kind, including at least breast, brain, lung, colon, liver, pancreatic, stomach, bone, skin, testicular, ovarian, endometrial, cervical, kidney, blood, brain, prostate, head and neck, spleen, thyroid, pituitary gland, and so forth. The cancer may be primary, metastatic, or refractory, for example. In particular aspects, fibroblasts are used as delivery compositions for one or more tumor inhibitory agents. The fibroblasts are modified to have one or more enhanced activities for use as a delivery agent, in at least certain aspects. The modified fibroblasts may be manipulated to comprise in and/or on the fibroblast cells to have one or more tumor inhibitory agents. In some cases the fibroblast cells are utilized with a carrier for the cells. In specific cases the fibroblasts are modified to alter gene expression of one or more genes that facilitate use as a delivery agent, such as being able to home to cancer cells/tissue (including improvement of an innate ability of fibroblasts to home to cancer cells/tissue, in at least some cases); such gene expression may include upregulation and/or downregulation of one or more particular genes and/or expression of one or more exogenously provided, recombinant genes, such as from an expression vector.

The invention provides an *in vitro* method of preparing fibroblasts as anti-cancer delivery compositions, comprising the steps of: (a) modifying fibroblasts to enhance activity of homing to cancer cells or tissue comprising cancer cells; and (b) manipulating fibroblasts to comprise one or more tumor inhibitory agents, wherein the fibroblasts of step (a), step (b), or both are exposed to hypoxia under suitable conditions and wherein the tumor inhibitory agent comprises a vector that expresses interleukin-12 (IL-12). In some aspects, step (a) occurs before step (b) whereas in other cases step (a) occurs after step (b). In some cases, step (a) and step (b) occur substantially concomitantly. In certain aspects, modifying fibroblasts to enhance activity of homing to cancer cells or tissue comprising cancer cells comprises transfecting the fibroblasts with one or more chemokine receptors. The chemokine receptor may be of any kind, including at least chemokine(C-X-C) receptor 4 (CXCR4) receptor. The hypoxia may be from 0.1%-10%, 0.1%-5%, 0.1%-2.5%, 0.1%-2%, 0.1%-1%, 0.5%-10%, 0.5%-7.5%, 0.5%-5%, 0.5%-2.5%, 0.5%-2%, 0.5%-1%, 1%-10%, 1%-7.5%, 1%-5%, 1%-2.5%, 1%-2%, 2%-10%, 2%-7.5%, 2%-5%, 2%-2.5%, 5%-10%, 5%-7.5%, 5%-6%, or 7.5%-10% oxygen, for example. The hypoxia may be exposed to the fibroblasts for a duration of at least or no more than or about 30 minutes (min)-3 days, 30 min-2 days, 30 min-1 day, 30 min-12 hours (hrs), 30 min-8 hrs, 30 min-6 hrs, 30 min-4 hrs, 30 min-2 hrs, 30 min-1 hr, 1 hr-3 days, 1hr-2 days, 1 hr-1 day, 1-12 hrs, 1-8 hrs, 1-6 hrs, 1-4 hrs, 1-2 hrs, 2 hrs-3 days, 2hrs-2 days, 2 hrs-1 day, 2 hrs-12 hrs, 2-10hrs, 2-8hrs, 2-6 hrs, 2-4 hrs, 2-3 hrs, 6 hrs-3 days, 6 hrs-2 days, 6 hrs-1 day, 6-12 hrs, 6-8 hrs, 8hrs-3 days, 8 hrs-2 days, 8 hrs-1 day, 8-16 hrs, 8-12 hrs, 8-10 hrs, 12hrs-3 days, 12 hrs-2 days, 12 hrs-1 day, 12-18hrs, 12-14hrs, 1-3 days, or 1-2 days, for example. In some cases, the tumor inhibitory agent is a nucleic acid, protein, or peptide, or it may be a virus, such as an oncolytic virus, for example vaccinia virus. In some cases, the tumor inhibitory agent comprises a vector, such as one that expresses TNF-alpha, TNF-related apoptosis-inducing ligand (TRAIL), a suicide gene, thymidylate synthase, bone morphogenic protein 4 (BMP4), HSV-thymidine kinase, an oncolytic adenovirus, interleukin-2 (IL-2), interleukin-12 (IL-12), interleukin-18 (IL-18), interleukin-23 (IL-23), Interferon-alpha, Interferon-beta, or a combination thereof. An effective amount of the modified fibroblasts may be delivered to an individual, including one that has cancer or is at risk for having cancer. The individual may be provided one or more additional anti-cancer agents, such as surgery, chemotherapy, radiation, hormone therapy, and/or immunotherapy. In some cases, the fibroblasts of step (a), step (b), or both are exposed to one or more histone deacetylase inhibitors and/or the fibroblasts of step (a), step (b), or both are exposed to one or more immunotherapeutic molecules. The immunotherapeutic molecule may be an antibody, such as a bispecific antibody.

In one aspect, there is an isolated fibroblast comprising: (a) an oncolytic virus; (b) a vector that encodes one or more tumor inhibitory agents; or (c) both. In another aspect there is a population of isolated fibroblasts comprising: (a) an oncolytic virus; (b) a vector that encodes one or more tumor inhibitory agents; or (c) both.

### BRIEF DESCRIPTION OF THE DRAWINGS

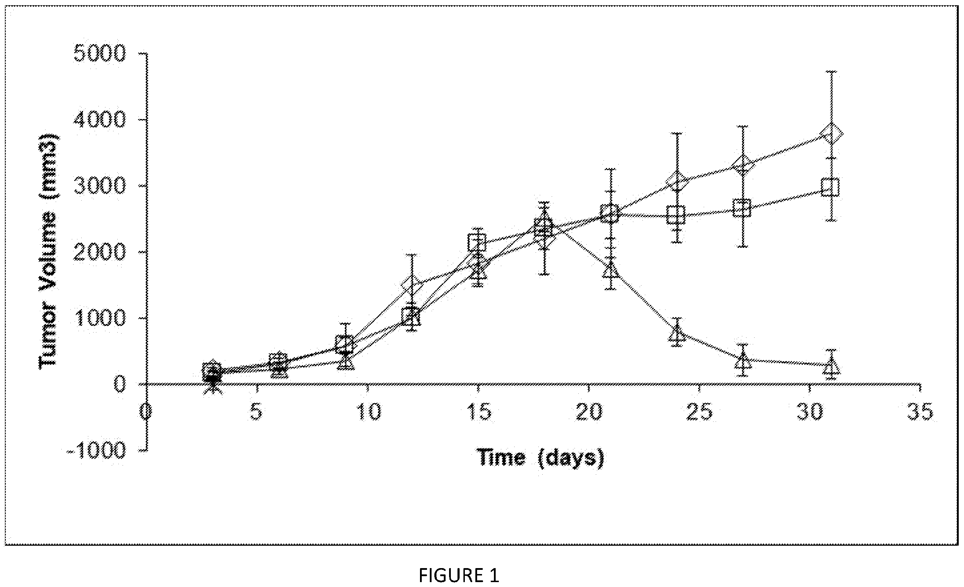

FIG. 1 shows significant inhibition of growing tumor in mice administered fibroblasts transfected with Newcastle disease virus. Female C57B6 Mice (10 per group) were treated with saline (diamond), uninfected fibroblasts, 100,000 cells administered intravenously, (square), or Newcastle disease virus infected fibroblasts (triangle) until day 31 .

### DETAILED DESCRIPTION

### I. Examples of Definitions

In keeping with long-standing patent law convention, the words "a" and "an" when used in the present specification in concert with the word comprising, including the claims, denote "one or more." Some aspects of the disclosure may consist of or consist essentially of one or more elements, method steps, and/or methods of the disclosure. It is contemplated that any method or composition described herein can be implemented with respect to any other method or composition described herein.

As used herein, the term "about" or "approximately" refers to a quantity, level, value, number, frequency, percentage, dimension, size, amount, weight or length that varies by as much as 30, 25, 20, 25, 10, 9, 8, 7, 6, 5, 4, 3, 2 or 1 % to a reference quantity, level, value, number, frequency, percentage, dimension, size, amount, weight or length. In particular aspects, the terms "about" or "approximately" when preceding a numerical value indicates the value plus or minus a range of 15%, 10%, 5%, or 1%. With respect to biological systems or processes, the term can mean within an order of magnitude, preferably within 5-fold, and more preferably within 2-fold, of a value. Unless otherwise stated, the term 'about' means within an acceptable error range for the particular value.

The term "administered" or "administering", as used herein, refers to any method of providing a composition to an individual such that the composition has its intended effect on the patient. For example, one method of administering is by an indirect mechanism using a medical device such as, but not limited to a catheter, applicator gun, syringe *etc.* A second exemplary method of administering is by a direct mechanism such as, local tissue administration, oral ingestion, transdermal patch, topical, inhalation, suppository *etc.*

As used herein, "allogeneic" refers to tissues or cells from another body that in a natural setting are immunologically incompatible or capable of being immunologically incompatible, although from one or more individuals of the same species.

As used herein, the term "allotransplantation" refers to the transplantation of organs, tissues, and/or cells from a donor to a recipient, where the donor and recipient are different individuals, but of the same species. Tissue transplanted by such procedures is referred to as an allograft or allotransplant.

As used herein, the terms "allostimulatory" and "alloreactive" refer to stimulation and reaction of the immune system in response to an allologous antigens, or "alloantigens" or cells expressing a dissimilar HLA haplotype.

As used herein, "autologous" refers to tissues or cells that are derived or transferred from the same individual's body (*i.e.,* autologous blood donation; an autologous bone marrow transplant).

As used herein, the term "autotransplantation" refers to the transplantation of organs, tissues, and/or cells from one part of the body in an individual to another part in the same individual, *i.e.,* the donor and recipient are the same individual. Tissue transplanted by such "autologous" procedures is referred to as an autograft or autotransplant.

The term "biologically active" refers to any molecule having structural, regulatory or biochemical functions. For example, biological activity may be determined, for example, by restoration of wild-type growth in cells lacking protein activity. Cells lacking protein activity may be produced by many methods (*i.e.,* for example, point mutation and frame-shift mutation). Complementation is achieved by transfecting cells that lack protein activity with an expression vector that expresses the protein, a derivative thereof, or a portion thereof. In other cases, a fragment of a gene product (such as a protein) may be considered biologically active (or it may be referred to as functionally active) if it retains the activity of the full-length gene product, although it may be at a reduced but detectable level of the activity of the full-length gene product.

"Cell culture" is an artificial *in vitro* system containing viable cells, whether quiescent, senescent or (actively) dividing. In a cell culture, cells are grown and maintained at an appropriate temperature, typically a temperature of 37°C and under an atmosphere typically containing oxygen and CO₂, although in other cases these are altered. Culture conditions may vary widely for each cell type though, and variation of conditions for a particular cell type can result in different phenotypes being expressed. The most commonly varied factor in culture systems is the growth medium. Growth media can vary in concentration of nutrients, growth factors, and the presence of other components. The growth factors used to supplement media are often derived from animal blood, such as calf serum.

Throughout this specification, unless the context requires otherwise, the words "comprise", "comprises" and "comprising" will be understood to imply the inclusion of a stated step or element or group of steps or elements but not the exclusion of any other step or element or group of steps or elements. By "consisting of" is meant including, and limited to, whatever follows the phrase "consisting of." Thus, the phrase "consisting of" indicates that the listed elements are required or mandatory, and that no other elements may be present. By "consisting essentially of' is meant including any elements listed after the phrase, and limited to other elements that do not interfere with or contribute to the activity or action specified in the disclosure for the listed elements. Thus, the phrase "consisting essentially of' indicates that the listed elements are required or mandatory, but that no other elements are optional and may or may not be present depending upon whether or not they affect the activity or action of the listed elements.

The term "drug", "agent" or "compound" as used herein, refers to any pharmacologically active substance capable of being administered that achieves a desired effect. Drugs or compounds can be synthetic or naturally occurring, non-peptide, proteins or peptides, oligonucleotides, or nucleotides (DNA and/or RNA), polysaccharides or sugars.

The term "individual", as used herein, refers to a human or animal that may or may not be housed in a medical facility and may be treated as an outpatient of a medical facility. The individual may be receiving one or more medical compositions *via* the internet. An individual may comprise any age of a human or non-human animal and therefore includes both adult and juveniles (*i.e.,* children) and infants. It is not intended that the term "individual" connote a need for medical treatment, therefore, an individual may voluntarily or involuntarily be part of experimentation whether clinical or in support of basic science studies. The individual may have one or more symptoms of a medical condition or may be asymptomatic. The term "subject" or "individual" refers to any organism or animal subject that is an object of a method or material, including mammals, *e.g.,* humans, laboratory animals (*e.g.,* primates, rats, mice, rabbits), livestock (*e.g.,* cows, sheep, goats, pigs, turkeys, and chickens), household pets (*e.g.,* dogs, cats, and rodents), horses, and transgenic non-human animals.

Reference throughout this specification to "one aspect," "an aspect," "a particular aspect," "a related aspect," "a certain aspect," "an additional aspect," or "a further aspect" or combinations thereof means that a particular feature, structure or characteristic described in connection with the aspect is included in at least one aspect of the present disclosure. Thus, the appearances of the foregoing phrases in various places throughout this specification are not necessarily all referring to the same aspect. Furthermore, the particular features, structures, or characteristics may be combined in any suitable manner in one or more aspects.

The term "pharmaceutically" or "pharmacologically acceptable", as used herein, refer to molecular entities and compositions that do not produce adverse, allergic, or other untoward reactions when administered to an animal or a human.

The term, "pharmaceutically acceptable carrier", as used herein, includes any and all solvents, or a dispersion medium including, but not limited to, water, ethanol, polyol (for example, glycerol, propylene glycol, and liquid polyethylene glycol, and the like), suitable mixtures thereof, and vegetable oils, coatings, isotonic and absorption delaying agents, liposome, commercially available cleansers, and the like. Supplementary bioactive ingredients also can be incorporated into such carriers.

The terms "reduce," "inhibit," "diminish," "suppress," "decrease," "prevent" and grammatical equivalents (including "lower," "smaller," *etc.*) when in reference to the expression of any symptom in an untreated subject relative to a treated subject, mean that the quantity and/or magnitude of the symptoms in the treated subject is lower than in the untreated subject by any amount that is recognized as clinically relevant by any medically trained personnel. In one aspect, the quantity and/or magnitude of the symptoms in the treated subject is at least 10% lower than, at least 25% lower than, at least 50% lower than, at least 75% lower than, and/or at least 90% lower than the quantity and/or magnitude of the symptoms in the untreated subject.

"Therapeutic agent" means to have "therapeutic efficacy" in modulating angiogenesis and/or wound healing and an amount of the therapeutic is said to be a "angiogenic modulatory amount", if administration of that amount of the therapeutic is sufficient to cause a significant modulation (*i.e.,* increase or decrease) in angiogenic activity when administered to a subject (e.g., an animal model or human patient) needing modulation of angiogenesis.

As used herein, the term "therapeutically effective amount" is synonymous with "effective amount", "therapeutically effective dose", and/or "effective dose" and refers to the amount of compound that will elicit the biological, cosmetic or clinical response being sought by the practitioner in an individual in need thereof. As one example, an effective amount is the amount suitable to enhance activity of homing to cancer cells or tissue comprising cancer cells or an effective amount is the ability to act as anti-cancer delivery compositions. The delivery may be local or systemic. As one example, an effective amount is the amount sufficient to reduce immunogenicity of a group of cells. The appropriate effective amount to be administered for a particular application of the disclosed methods can be determined by those skilled in the art, using the guidance provided herein. For example, an effective amount can be extrapolated from *in vitro* and *in vivo* assays as described in the present specification. One skilled in the art will recognize that the condition of the individual can be monitored throughout the course of therapy and that the effective amount of a compound or composition disclosed herein that is administered can be adjusted accordingly.

As used herein, the term "transplantation" refers to the process of taking living tissue or cells and implanting it in another part of the body or into another body.

"Treatment," "treat," or "treating" means a method of reducing the effects of a disease or condition. Treatment can also refer to a method of reducing the disease or condition itself rather than just the symptoms. The treatment can be any reduction from pre-treatment levels and can be but is not limited to the complete ablation of the disease, condition, or the symptoms of the disease or condition. Therefore, in the disclosed methods, treatment" can refer to a 10%, 20%, 30%, 40%, 50%, 60%, 70%, 80%, 90%, or 100% reduction in the severity of an established disease or the disease progression, including reduction in the severity of at least one symptom of the disease. For example, a disclosed method for reducing the immunogenicity of cells is considered to be a treatment if there is a detectable reduction in the tumor load or improvement of at least one symptom when compared to pre-treatment levels in the same subject or control subjects. Thus, the reduction can be a 10, 20, 30, 40, 50, 60, 70, 80, 90, 100%, or any amount of reduction in between as compared to native or control levels. It is understood and herein contemplated that "treatment" does not necessarily refer to a cure of the disease or condition, but an improvement in the outlook of a disease or condition. In specific aspects, treatment refers to the lessening in severity or extent of at least one symptom and may alternatively or in addition refer to a delay in the onset of at least one symptom.

Disclosed are means, methods and compositions of matter useful for treatment of any cancer by leveraging and augmenting the ability of fibroblasts (autologous, xenogeneic, syngeneic, or allogeneic, as examples) to home to tumors. In one aspect, allogeneic fibroblasts are cultured under hypoxic conditions to enhance expression of one or more chemokine receptors useful for enhanced migration to tumor sites, although in optional cases the fibroblasts are not cultured under hypoxic conditions. The fibroblasts may be further modified themselves to deliver one or tumor inhibitory agents. A tumor inhibitory agent may be selected from a chemotherapeutic, an immunotherapeutic, a radiosensitizer, and/or stimulatory of hyperthermia, for example. The modified fibroblasts are subsequently administered by any suitable route, such as intravenously, intratumorally, or in a site into one or more tumors allowing for selective accumulation of the fibroblast cells.

### II. Fibroblasts, Methods of Preparing Same, and Methods of Modifying Same

In particular aspects fibroblasts are utilized as delivery agents for one or more tumor inhibitory agents (although the agent may also be utilized against non-solid cancers, such as hematological cancers). The fibroblasts may be modified to enhance their capability for use as delivery agents and then manipulated to comprise one or more anti-tumor agents to be delivered, although this order may be reversed, in specific aspects.

### A. Enhancement of Fibroblast Activity through Modification

In particular aspects the fibroblasts are modified prior to delivery to an individual in need of cancer treatment, such as modified to enhance their activity as delivery agents. Although the enhancement may be for any cellular activity, in specific aspects the enhancement allows the fibroblasts to home to cancer cells, cancerous tissue(s), or an organ that has cancer in at least part of it. In at least some cases, the fibroblasts are modified to have enhancement of an existing activity for homing to cancer cells or a cancer environment. In some cases the fibroblasts are modified before or after the fibroblasts are thus manipulated to harbor or comprise one or more tumor inhibitory agents. Although modification may occur by any suitable method, in some cases the fibroblasts are modified to enhance their ability to act as delivery agents for cancer treatment, including enhance their ability to home to cancer cells and/or cancerous tissue and/or an organ having cancer. In some cases, the fibroblasts are modified such that they have altered gene expression of one or more endogenous genes, including one or more genes being upregulated, one or more genes being downregulated, or both. In addition, or as an alternative, exogenous gene(s) may be added within the fibroblast cells, such as from an expression vector. In addition, or as an alternative, the fibroblasts are configured to be housed with a carrier, such as nanoparticles, and/or immunoisolatory sheaths that protect the cells from recipient immune rejection..

In particular aspects, fibroblasts are modified to enhance expression of one or more endogenous (and/or exogenously provided) chemokine receptors, including one or more receptors useful for enhanced migration of the fibroblasts to tumor sites. Examples of chemokine receptors include chemokine (C-X-C) receptor 4 (CXCR4) receptor, CXCL16 receptor and/or CCL9 receptor, and so forth. Thus, in specific cases the fibroblast cells are transfected with a vector that expresses one or more chemokine receptors (or functionally active fragments thereof) and these fibroblast cells are also manipulated to comprise one or more anti-tumor agents prior to delivery to an individual with cancer.

Although in some cases the fibroblast cells are modified through recombinant technology to upregulate gene expression of one or more chemokine receptors (such as transfected with an expression vector that encodes one or more chemokine receptors), in particular aspects the cells are modified through exposure of the fibroblasts cells to one or more particular agents and/or conditions. The exposure may occur by any suitable method, but in specific aspects the fibroblasts are cultured such that the cells are exposed to one or more particular agents and/or conditions. In specific aspects, the fibroblasts are exposed to hypoxia for a suitable duration of time, such as to increase expression of one or more chemokine receptor(s) or any other gene product involved in homing to cancers. The exposure to hypoxia for a particular duration of time may or may not be continuous. The exposure may be prior to manipulation of the fibroblast cells to comprise one or more tumor inhibitory agents and/or it may be subsequent to manipulation of the fibroblast cells to comprise one or more tumor inhibitory agents.

In particular aspects, the hypoxia condition comprises oxygen levels from 0.1%-10%, 0.1%-5%, 0.1%-2.5%, 0.1%-2%, 0.1%-1%, 0.5%-10%, 0.5%-7.5%, 0.5%-5%, 0.5%-2.5%, 0.5%-2%, 0.5%-1%, 1%-10%, 1%-7.5%, 1%-5%, 1%-2.5%, 1%-2%, 2%-10%, 2%-7.5%, 2%-5%, 2%-2.5%, 5%-10%, 5%-7.5%, 5%-6%, or 7.5%-10% oxygen, as examples only. The duration of exposure, including with (but not limited to) these representative levels of oxygen may be for a duration of 30 minutes (min)-3 days, 30 min-2 days, 30 min-1 day, 30 min-12 hours (hrs), 30 min-8 hrs, 30 min-6 hrs, 30 min-4 hrs, 30 min-2 hrs, 30 min-1 hour (hr), 1 hr-3 days, 1hr-2 days, 1 hr-1 day, 1-12 hrs, 1-8 hrs, 1-6 hrs, 1-4 hrs, 1-2 hrs, 2 hrs-3 days, 2hrs-2 days, 2 hrs-1 day, 2 hrs-12 hrs, 2-10hrs, 2-8hrs, 2-6 hrs, 2-4 hrs, 2-3 hrs, 6 hrs-3 days, 6 hrs-2 days, 6 hrs-1 day, 6-12 hrs, 6-8 hrs, 8hrs-3 days, 8 hrs-2 days, 8 hrs-1 day, 8-16 hrs, 8-12 hrs, 8-10 hrs, 12hrs-3 days, 12 hrs-2 days, 12 hrs-1 day, 12-18hrs, 12-14hrs, 1-3 days, or 1-2 days, as examples only.

In some aspects, fibroblasts are infected with oncolytic or tumor trophic viruses, including adenoviruses [9-14], vaccinia virus [15], herpes virus [16-20], reovirus [21], measles [22, 23], Newcastle Disease Virus [24-26]. Infected fibroblasts are used to deliver viral payload to tumor cells.

In particular aspects, the fibroblasts are modified to increase expression of one or more chemokine receptors and/or are transfected with recombinant one or more chemokine receptors.

### B. Manipulation of Fibroblasts as Delivery Agents

In particular aspects, in addition to having enhanced activity for homing to cancer cells, the fibroblasts are manipulated to act as delivery agents for one or more tumor inhibitory agents. The tumor inhibitory agent may be a nucleic acid (DNA or RNA), protein, peptide, and/or an oncolytic or tumor trophic virus, and so forth. In specific aspects, the fibroblasts are manipulated to harbor a vector that produces a gene product (RNA itself or an expressed protein or peptide) that has tumor inhibitory properties. The fibroblasts may comprise an expression vector that encodes a therapeutic gene product, a chemotherapeutic agent, a hormone, an immunotherapeutic, or a combination thereof. When more than one recombinant tumor inhibitory agent is desired to be expressed from a vector, the multiple tumor inhibitory agents may or may not be expressed from the same vector.

In certain cases, a vector that expresses one or more cancer-inhibitory genes that have tumor inhibitory properties is transfected into the fibroblasts. The cancer(tumor)-inhibitory gene may be of any kind, but in specific aspects it is TNF-alpha, TRAIL, a suicide gene (that in specific aspects kills the fibroblast but kills the tumor cells also), thymidylate synthase, or a combination thereof. In addition, or alternative to this, an increased ability of fibroblasts to inhibit cancer may be endowed by transfection of the fibroblasts with one or more immune stimulatory genes. Examples of immune stimulatory genes include at least IL-2, IL-15, IL-22, IL-18, and/or IL-27. In addition, or alternatively, the fibroblasts are manipulated to express a bispecific antibody. Examples of bispecific antibodies useful for the methods and compositions of the disclosure include antibodies that bind to an immune molecule and/or additionally to a cancer cell. Useful examples including antibodies capable of binding concurrently to CD3 on T cells and MUC1 on tumor cells, or in another aspect bispecific antibodies bind to CD56 on NK cells and HER2 on cancer cells, or in another aspect bispecific antibodies bind to CD 16 on monocytes and membrane bound vimentin on cancer cells.

In specific aspects, persistence of the fibroblasts *in vivo* may be augmented through transfection of a therapeutic peptide sequence encoding an anti-apoptotic gene, such as one selected from a group consisting of obestatin, XIAP, survivin, BCL-2, BCL-XL, GATA-4, IGF-1, EGF, heme-oxygenase-1, NF-kB, akt, pi3-k, epha-2, and a combination thereof.

In certain aspects, the fibroblasts are modified to increase their persistence *in vivo.* Although this may occur by any suitable means, in specific aspects persistence of fibroblasts *in vivo* is augmented through transfection of a gene construct capable of inducing RNA interference directed against a molecule associated with induction of apoptosis, such as one or more selected from the group consisting of: Fas, FasL, CASP1 (ICE), CASP10 (MCH4), CASP14, CASP2, CASP3, CASP4, CASP5, CASP6, CASP7, CASP8, CASP9, CFLAR (CASPER), CRADD, PYCARD (TMS1/ASC), ABL1, AKT1, BAD, BAK1, BAX, BCL2L11, BCLAF1, BID, BIK, BNIP3, BNIP3L, CASP1 (ICE), CASP10 (MCH4), CASP14, CASP2, CASP4, CASP6, CASP8, CD70 (TNFSF7), CIDEB, CRADD, FADD, FASLG (TNFSF6), HRK, LTA (TNFB), NOD1 (CARD4), PYCARD (TMS1/ASC), RIPK2, TNF, TNFRSF10A, TNFRSF10B (DR5), TNFRSF25 (DR3), TNFRSF9, TNFSF10 (TRAIL), TNFSF8, TP53, TP53BP2, TRADD, TRAF2, TRAF3, TRAF4, and a combination thereof.

In particular aspects, modified fibroblasts are endowed with an ability to differentiate into cells of the neuronal lineage at a specified time point associated with fibroblast homing to neuronal tissue.

In particular aspects, modified fibroblasts are manipulated to have the ability to differentiate into certain lineages. In particular aspects, the modified fibroblasts are manipulated to differentiate into a neural lineage. In specific aspects, differentiation into the neural lineage is accomplished by transfection of one or more therapeutic polynucleotides that encode polypeptide sequences selected from the group of polypeptide sequences consisting of ADCYAP1R1, ARTN, BDNF, CD40 (TNFRSF5), CNTF, CNTFR, CRHBP, CRHR1, CRHR2, FRS2, FRS3, FUS, GDNF, GFRA1, GFRA2, GFRA3, GMFB, GMFG, MAGED1, MT3, NF1, NGF, NGFR, NGFRAP1, NR1I2, NRG1, NRG2, NTF3, NTF4, NTRK1, NTRK2, PSPN, PTGER2, TFG, TRO, VGF, and a combination thereof.

In some aspects, one or more polypeptide sequences are utilized to induce differentiation of endogenous neural progenitors. In some cases, one or more therapeutic polypeptide sequences are transfected to increase mobility of the fibroblasts. In specific aspects, mobility of fibroblast refers to chemotaxis to an area of hypoxia, and in certain cases, mobility of fibroblast refers to chemotaxis to an area of inflammation.

In one aspect of the disclosure, transfected fibroblasts have enhanced homing activity to tumors because of exogenous nucleic acids therein that cause cytototoxicity or inhibition of tumor growth. In some aspects the nucleic acid(s) induce an immune response. Transfection methodologies used for administration of gene modified mesenchymal stem cells (for example) may be applied to fibroblasts for the practice of the disclosure. For example, fibroblasts may be transfected with prodrugs [1]. One particular aspect includes introducing a suicide gene [2-5], such as cytosine deaminase::uracil phosphoribosyltransferase (CD::UPRT), to convert the relatively nontoxic 5-fluorocytosine (5-FC) into the highly toxic antitumor 5-fluorouracil (5-FU) [6-8]. In other aspects, fibroblasts are used for delivery of oncolytic or tumor trophic viruses, including adenoviruses [9-14], vaccinia virus [15], herpes virus [16-20], reovirus [21], measles [22, 23], Newcastle Disease Virus [24-26],

Transfection of immunogenic cytokines into fibroblasts may be employed to possess enhanced tumor homing ability. Examples of cytokines include TRAIL [27-36], TNF-alpha [37], interferon gamma [38], interferon alpha [39], interferon beta [40-43], IL-12 [44, 45], IL-18 [46, 47], IL-21 [48], and IL-28 [49], for example.

Immunotherapeutic molecules such as bispecific antibodies may be utilized in the fibroblasts to activate immune cells in the proximity of the tumor [50]. Immuno-apoptins may also be utilized for transfection [51].

Modified fibroblasts may express one or more nucleic acid molecules for use in the methods of the presently disclosed subject matter, including those that encode one or more bioactive molecules functional in the treatment of an oncological indication. The one or more bioactive molecules may be selected from the group consisting of proteins, polypeptides, peptides, drugs, enzymes, hormones, RNA, and metabolites. In a particular aspect, the cancer is a brain tumor, and the one or more bioactive molecules comprise one or more anti-cancer agents, particularly wherein the one or more anti-cancer agents are selected from the group consisting of bone morphogenic protein 4 (BMP4), TNF-related apoptosis-inducing ligand (TRAIL), HSV-thymidine kinase, an oncolytic adenovirus, interleukin-2 (IL-2), interleukin-12 (IL-12), interleukin-18 (IL-18), interleukin-23 (IL-23), Interferon-alpha, and Interferon-beta.

A molecule for use in the methods of the presently disclosed subject matter may encode one or more bioactive molecules functional in the treatment of a neurological disease, for example. The one or more bioactive molecules may be selected from the group consisting of proteins, polypeptides, peptides, drugs, enzymes, hormones, RNA, and metabolites. In a particular aspect, the neurological disease is a brain tumor, and the one or more bioactive molecules comprise one or more anti-cancer agents, such as wherein the one or more anti-cancer agents are selected from the group consisting of bone morphogenic protein 4 (BMP4), TNF-related apoptosis-inducing ligand (TRAIL), HSV-thymidine kinase, an oncolytic adenovirus, interleukin-2 (IL-2), interleukin-12 (IL-12), interleukin-18 (IL-18), interleukin-23 (IL-23), Interferon-alpha, and Interferon-beta.

In any case wherein a fibroblast encompasses a vector that expresses one or more cancer(tumor)-inhibitory genes, the vector may be a) a lentiviral vector; b) adenoviral vector; c) an adeno-associated viral vector; or d) a retroviral vector. In particular aspects, any of the exogenously provided cancer(tumor)-inhibitory genes may in specific aspects be under control of one or more inducible promoters (for example, a rheoswitch). In some cases, any promoter utilized in vectors in the fibroblasts may be constitutive, inducible, tissue-specific, or a combination thereof, for example.

In some aspects, an increased ability of the fibroblasts to inhibit cancer is accomplished by infection of the fibroblasts with an oncolytic virus, wherein the oncolytic virus is the tumor inhibitory agent. In such cases, the fibroblast manufactures high concentrations of the virus, and the fibroblast cells can be delivered directly to the tumor itself (such as by injection) or in the environment of the tumor.

Although any oncolytic virus may be utilized, in some cases the oncolytic virus may be selected from the group consisting of: a) vaccinia virus; b) reovirus; c) Newcastle Disease Virus; and d) herpes virus, for example. In specific aspects, the oncolytic viruses are configured to express one or more tumor inhibitory agent gene products themselves.

In some cases, a fibroblast comprises an oncolytic virus but does not comprise any other modifications, such as does not express an endogenous chemokine receptor and has not been exposed to hypoxia. In specific cases, a fibroblast relies on innate capabilities of homing to cancer cells yet comprises an oncolytic virus as an tumor inhibitory agent. Fibroblasts may consist of a manipulation by the hand of man to harbor an oncolytic virus.

Accordingly, in some aspects, *in situ* vaccination comprises delivering (such as by injecting) modified fibroblasts into the individual, wherein the modified fibroblast comprises a cytotoxic payload. In some aspects, the modified fibroblasts additionally or alternatively comprise one or more imaging payloads. In some aspects, the modified fibroblast comprises one or more of a virus, an antibody, and/or a cytokine as the cytotoxic payload. In some aspects, the modified fibroblast expresses a cytokine as the cytotoxic payload. In some aspects, the cytokine is selected from colony-stimulating factor (CSF), interferon (IFN), interleukin (IL), stem cell factor (SCF), tumor growth factors (TGF), and tumor necrosis factor (TNF). In some aspects, the cytokine is a colony stimulating factor (CSF). In some aspects, the CSF is Granulocyte-colony stimulating factor (G-CSF), macrophage colony-stimulating factor (M-CSF), or Granulocyte-macrophage colony-stimulating factor (GM-CSF). In some aspects, the CSF is selected from ancestim, garnocestim, pegacaristim, leridistim, milodistim, filgrastim, lenograstim, nartograstim, pegfilgrastim, pegnartograstim, ecogramostim, molgramostim, regramostim, sargramostim, cilmostim, lanimostim, mirimostim, daniplestim, muplestim, or derivativees thereof. In some aspects, the cytokine is an interleukin (IL). In some aspects, the interleukin is selected from IL-1, IL-2, IL-3, IL-4, IL-5, IL-6, IL-7, IL-8, IL-9, IL-10, IL-11, IL-12, IL13, IL-14, IL-15, IL-16, IL-17, IL-18, IL-19, IL-20, IL-21, IL-22, IL-23, IL-24, IL-25, IL-26, IL-27, IL-28, IL-29, IL-30, IL-31, IL-32, IL-34. IL-35, and derivatives thereof. In some aspects, the interleukin is IL-2, IL-4, or derivatives thereof. In some aspects, the cytotoxic payload comprises a lytic virus. In some aspects, the lytic virus is a vaccinia virus. In some aspects, the cytotoxic payload comprises one or more chemotherapeutic agents. In some aspects, delivery of the fibroblasts encompassed by the disclosure results in in situ vaccination of the individual against the tumor.

In some aspects, the modified fibroblast is an adult fibroblast. In some aspects, the modified fibroblast is transformed with a lentivirus or retrovirus. In some aspects, the modified fibroblast is transiently transfected with an artificial chromosome, virus and/or plasmid DNA. In some aspects, the modified fibroblast is capable of localizing to the tumor. In some aspects, the modified fibroblast is autologous with respect to a recipient individual. In some aspects, the modified fibroblast is allogeneic with respect to a recipient individual. In some aspects, the modified fibroblast is selected from the group consisting of adult fibroblasts, embryonic fibroblasts, fetal fibroblasts, placental fibroblasts, adipose stromal derived fibroblasts, and combinations thereof. In some aspects, the modified cell is derived from adipose-derived Stromal Vascular Fraction (SVF) fibroblasts.

Other agents may be used within the practice of the current disclosure to augment immune modulatory, migratory, or growth factor-producing activity of the modified fibroblasts, which include, a) a TLR agonist; b) intravenous immunoglobulin (IVIG); c) monocyte conditioned media; d) supernatant from neutrophil extracellular trap exposed peripheral blood mononuclear cells; e) co-culture with monocytes; f) co-culture with monocytes that have been pretreated with IVIG; g) co-culture with T cells; h) co-culture with T cells that have been exposed to a T cell stimulus; i) co-culture with NK cells; j) peptidoglycan isolated from gram positive bacteria; k) lipoteichoic acid isolated from gram positive bacteria; 1) lipoprotein isolated from gram positive bacteria; m) lipoarabinomannan isolated from mycobacteria, n) zymosan isolated from yeast cell well; o) Polyadenylic-polyuridylic acid; p) poly (IC); q) lipopolysaccharide; r) monophosphoryl lipid A; s) flagellin; t) Gardiquimod; u) Imiquimod; v) R848; w) oligonucleosides containing CpG motifs; and/or x) 23S ribosomal RNA.

In some aspects, upon delivery (such as by injection) to an individual of modified fibroblasts infected with an oncolytic virus or a plurality of oncolytic viruses results in induction of a T-cell response to tumor antigens in the individual.

In some aspects, the fibroblast is an adult fibroblast. In some aspects, the fibroblast is capable of excreting one or more growth factors. In some aspects, the fibroblast produces one or more antibodies and/or one or more growth factors capable of stimulating T-cell growth and expansion. In some aspects, the fibroblast is transformed with a lentivirus or retrovirus. In some aspects, the fibroblast is transiently transfected with an artificial chromosome, virus or plasmid DNA. In some aspects, the lentivirus or retrovirus comprise a heterologous nucleic acid encoding a protein involved in T-cell activation. In some aspects, the adult fibroblasts are permanently transformed (e.g. with lentivirus or retrovirus), or transiently altered with artificial chromosomes, viruses or plasmid DNA, which results in the production of one or more antibodies, one or more growth factors, and/or one or more other proteins as payloads that stimulate T-cell growth and expansion. In some aspects, the fibroblast is injected into a site of the tumor. In some aspects, the fibroblast is injected into the tumor.

In specific aspects, the fibroblasts are produced and housed in a repository prior to use. As such, the fibroblasts may be utilized in an off-the-shelf manner for a recipient individual. In some cases, the housed fibroblasts undergo further modification after being obtained from the repository to tailor the fibroblasts to a recipient individual, whereas in other cases they are not further modified.

### III. Methods of Use

In particular aspects, fibroblasts encompassed by the disclosure are provided in suitable amounts to an individual with cancer to treat the cancer, including to alleviate at least one symptom thereof. The modified fibroblasts may be provided to an individual in effective amounts once or more than once. In some cases when they are provided more than once to an individual they are modified in substantially the same way (as an example, to comprise the same tumor inhibitory agent(s)) and in certain cases the fibroblasts of subsequent administrations are obtained from the same population of modified fibroblasts as earlier administration(s). In other cases a new population of fibroblasts are modified in substantially the same way as earlier administrations but are obtained from a different population of fibroblasts. In some cases, when fibroblasts are provided more than once to an individual, they are modified differently as fibroblasts of earlier administration(s). For example, in some cases subsequent administrations of fibroblasts comprise a different tumor inhibitory agent(s) as earlier administrations. In specific cases, the individual is provided a different tumor inhibitory agent(s) because the individual has developed resistance to the earlier tumor inhibitory agent(s).

When the fibroblasts are provided to an individual, they may be administered in any suitable route. The fibroblasts may be provided to the individual locally or systemically. In some cases the fibroblasts are administered into a tumor and/or in a tumor microenvironment and/or near a tumor site. When multiple tumors are present in an individual there may be local deliveries to all of the tumors or a subset of the tumors. In particular aspects, fibroblasts are administered intravenously, intrathecally, intraventricularly, intramuscularly, subcutaneously intrarectally, intra-omentally, intrahepatically, topically, intrarenally, by inhalation, or orally, for example.

In particular aspects, the methods comprise the step of delivering an effective amount of fibroblasts to an individual in need thereof, wherein the fibroblasts comprise the ability to hone to cancer and/or comprise one or more tumor inhibitory agents. As part of the method, production of the fibroblasts to have this or these abilities is part of the method, although in some cases it is not. In specific aspects, methods are provided for treating any type of cancer in an individual, comprising: a) obtaining or selecting a fibroblast population; b) increasing ability of the fibroblasts to home to a tumor microenvironment; c) increasing the ability of the fibroblasts to inhibit cancer; and d) administering the fibroblasts to an individual in need of treatment. In specific aspects, the increased ability of the fibroblasts to home to tumors comprises incubation of the fibroblast in a hypoxic environment, such as from 0.1% oxygen to 10% oxygen for a period of 30 minutes to 3 days, for example. The fibroblast may be cultured under specific conditions, such as at 3% oxygen for 24 hours, in specific aspects.

In one aspect of the disclosure, treated fibroblasts that are transfected with the nucleic acid molecules may also be administered to the patient in combination with an additional therapeutic agent and/or treatment, including radiotherapy, chemotherapy, surgery, hormone therapy, immunotherapy, or a combination thereof, for example. Additional cancer therapeutic agents may also include, but are not limited to chemotherapeutic agent(s) such as adriamycin, dexamethasone, vincristine, cyclophosphamide, fluorouracil, topotecan, taxol, interferons, platinum derivatives or a combination thereof. Other examples of agents with which the disclosed fibroblasts transfected with the nucleic acid molecules may also be administered include, without limitation, anti-inflammatory agents such as corticosteroids, TNF blockers, IL-I RA, azathioprine, cyclophosphamide, and sulfasalazine; immunomodulatory and immunosuppressive agents such as cyclosporin, tacrolimus, rapamycin, mycophenolate mofetil, interferons, corticosteroids, cyclophophamide, azathioprine, and sulfasalazine; neurotrophic factors, such as acetylcholinesterase inhibitors, MAO inhibitors, interferons, anti-convulsants, ion channel blockers, riluzole, and antiparkinsonian agents; agents for treating cardiovascular disease such as beta-blockers, ACE inhibitors, diuretics, nitrates, calcium channel blockers, and statins; agents for treating liver disease such as corticosteroids, cholestyramine, interferons, and anti-viral agents; agents for treating blood disorders, such as corticosteroids, anti-leukemic agents, and growth factors; agents for treating diabetes such as insulin, insulin analogues, alpha glucosidase inhibitors, biguanides, and insulin sensitizers; and agents for treating immunodeficiency disorders such as gamma globulin.

In particular aspects the fibroblasts comprise oncolytic viruses. That is, augmentation of efficacy of oncolytic viral therapy is achieved by infecting fibroblasts or fibroblasts with enhanced onco-migratory properties with oncolytic virus. Examples of use of oncolytic viruses has previously been used in the literature. For example, attenuated (non-pathogenic) avian viruses have been used as a form of non-specific immunological treatment for advanced human cancer (see [52], for example). The disclosure encompasses the augmentation of viral therapy/immunotherapy of cancer by selectively delivering virus to the tumor using modified fibroblasts.

In some aspects, chemotherapy is used to reduce tumor volume and/or increase immunogenicity of tumors. In some aspects, the treatment that will induce apoptosis in cells within the tumor comprises administration of a chemotherapeutic compound. In some aspects, the chemotherapeutic compound is given with the fibroblasts, whereas in other aspects the chemotherapeutic compound is incorporated into the fibroblasts by use of microbubbles that are loaded into the fibroblasts (Otani et al., 2009). Chemotherapeutic compounds include, but are not limited to platinum; platinum analogs (e.g., platinum coordination complexes) such as cisplatin, carboplatin, oxaliplatin, DWA2114R, NK121, IS 3 295, and 254-S; anthracenediones; vinblastine; alkylating agents such as thiotepa and cyclosphosphamide; alkyl sulfonates such as busulfan, improsulfan and piposulfan; aziridines such as benzodopa, carboquone, meturedopa and uredopa; ethylenimines and methylamelamines including altretamine, triethylenemelamine, trietylenephosphoramide, triethylenethiophosphaoramide and trimethylolomelamime nitrogen mustards such as chiorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, ranimustine; antibiotics such as aclacinomysins, actinomycin, authramycin, azaserine, bleomycins, cactinomycin, calicheamicin, carabicin, carminomycin, carzinophilin, chromomycins, dactinomycin, daunorubicin, detorubicin, 6-diazo-5-oxo-L-norleucine, doxorubicin, epirubicin, esorubicin, idarubicin, marcellomycin, mitomycins, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; amsacrine; bestrabucil; bisantrene; edatraxate; defofamine; demecolcine; diaziquone; elfornithine; elliptinium acetate; etoglucid; gallium nitrate; substituted ureas; hydroxyurea; lentinan; lonidamine; mitoguazone; mitoxantrone; mopidamol; nitracrine; pentostatin; phenamet; pirarubicin; podophyllinic acid; 2-ethylhydrazide; procarbazine; anti-cancer polysaccharides; polysaccharide-K; razoxane; sizofiran; spirogermanium; tenuazonic acid; triaziquone; 2,2',2"-trichlorotriethylamine; urethan; vindesine; dacarbazine; mannomustine; mitobronitol; mitolactol; pipobroman; gacytosine; cytosine arabinoside; cyclophosphamide; thiotepa; taxoids, such as paclitaxel and doxetaxel; chlorambucil; gemcitabine; 6-thioguanine; mercaptopurine; methotrexate; etoposide (VP-16); ifosfamide; mitomycin C; mitoxantrone; vincristine; vinorelbine; navelbine; novantrone; teniposide; daunomycin; aminopterin; XELODA; ibandronate; CPT11; topoisomerase inhibitor RFS 2000; difluoromethylornithine (DMFO); retinoic acid; esperamicins; capecitabine; methylhydrazine derivatives; Erlotinib (TARCEVA); sunitinib malate (SUTENT); and pharmaceutically acceptable salts, acids or derivatives of any of the above. Also included in this definition are anti-hormonal agents that act to regulate or inhibit hormone action on tumors such as anti-estrogens including, for example, tamoxifen, raloxifene, aromatase inhibiting 4(5)-imidazoles, 4-hydroxytamoxifen, trioxifene, keoxifene, LY117018, onapristone and toremifene (FARESTON); adrenocortical suppressants; and antiandrogens such as flutamide, nilutamide, bicalutamide, leuprolide and goserelin; and pharmaceutically acceptable salts, acids or derivatives of any of the above. Such chemotherapeutic compounds that can be used herein include compounds whose toxicities preclude use of the compound in general systemic chemotherapeutic methods. In some aspects, the chemotherapy comprises administration of a chemotherapeutic agent is selected from an alkylating drug, an antimetabolite, an antimytotic cytostatic, a topoisomerase inhibitor, antitumor antibiotic, and any other cytostatic, and/or a radiotherapy. In some aspects, the chemotherapeutic agent is an alkylating agent. In some aspects, the alkylating agent is selected from cisplatin, oxaliplatin, cyclop hosphamid, ifosfamid, trofosfamid, melphalan, chlorambucil, estramustin, busulfan, treosulfan, carmustin, lomustin, nimustin, streptozocin, procarbazin, dacarbazin, temozolomid, and thiotepa. In some aspects, the chemotherapeutic agent is an antimetabolite. In some aspects, the antimetabolite is selected from 5-fluorouracil, methotrexate, azacitidin, capecitabin, doxifluridin, cytarabin, gemcitabin, 6-thioguanin, pentostatin, azathioprin, 6-mercaptopurin, fludarabin, and cladribin. In some aspects, the chemotherapeutic agent is a topoisomerase inhibitor. In some aspects, the topoisomerase inhibitor is selected from doxorubicin, camptothecin, topotecan, irinotecan, etoposide, and teniposide. In some aspects, the chemotherapeutic agent is an antitumor antibiotic. In some aspects, the antitumor antibiotic is selected from tamoxifen, 5-fluoro-5'-deoxyuridine, belomycin, actinomycin D, and mitomycin. In some aspects, the chemotherapeutic agent is a cytostatic. In some aspects, the cytostatic is L-asparaginase or hydroxycarb amide.

In particular aspects, the fibroblasts are engineered to comprise a polynucleotide that encodes a gene switch. In at least certain cases of the gene switch aspect, a mammal exhibits antitumor immunity after the modified fibroblast cells and a ligand are administered to the mammal. In some cases, the fibroblast cells harbor a therapeutic polypeptide sequence that is selected based on a desired therapeutic effect of the modified fibroblasts.

In particular aspects of compositions of the disclosure, there is an *in vitro* engineered fibroblast comprising a vector comprising a polynucleotide encoding a gene switch, and the gene switch comprises (1) at least one transcription factor sequence, wherein the at least one transcription factor sequence encodes a ligand-dependent transcription factor comprising an ecdysone receptor ligand binding domain, operably linked to a promoter, and (2) a polynucleotide encoding a polypeptide at least 85% identical to a wild type human therapeutic polypeptide sequence linked to a promoter that is activated by the ligand-dependent transcription factor, wherein following administration of the in vitro-engineered fibroblast to a mammal with a disease and a first administration of a ligand to the mammal less than 48 hours after the in vitro fibroblasts are administered, wherein optionally the ligand is thereafter administered daily for a period of 2 to 30 days, the therapeutic effect of the engineered fibroblast in the mammal is reduced. In specific aspects, the polynucleotide encoding a gene switch comprises a first transcription factor sequence and a second transcription factor sequence under the control of a promoter, wherein the proteins encoded by the first transcription factor sequence and the second transcription factor sequence interact to form a protein complex that functions as a ligand-dependent transcription factor. The first transcription factor and the second transcription factor may be connected by an internal ribosomal entry site, in at least some cases. In specific aspects, the polynucleotide encoding a gene switch comprises a first transcription factor sequence under the control of a first promoter and a second transcription factor sequence under the control of a second promoter, wherein the proteins encoded by the first transcription factor sequence and the second transcription factor sequence interact to form a protein complex that functions as a ligand-dependent transcription factor.

Ligands related to the gene switch system may be of any specific kind, but in specific aspects the ligand is selected from the group consisting of RG-115819, RG-115932, and RG-115830. The ligand may be an amidoketone or oxadiazoline. In specific aspects, the ligand is administered less than one hour (or less than 2, 3, 4, 5, 6, 7, 8, 9, 10, 11, 12, 13, 14, 15, 16, 17, 18, 19, 20, 21, 22, 23, or 24 hours) before or after the in vitro-engineered fibroblasts are administered to the individual. The fibroblasts may be autologous or allogeneic to the individual.

In specific aspects, a polynucleotide sequence encoding a gene switch comprises a polynucleotide sequence encoding a VP-16 transactivation domain or a GAL-4 DNA binding domain.

In specific aspects, an ecdysone receptor ligand binding domain comprises a substitution mutation. The ecdysone receptor ligand binding domain may comprise a Choristoneura fumiferana ecdysone receptor ligand binding domain.

In some aspects, a polynucleotide sequence encoding a gene switch comprises a polynucleotide sequence encoding an RXR ligand binding domain, such as one selected from the group consisting of a vertebrate RXR ligand binding domain, an invertebrate RXR ligand binding domain, and a chimeric RXR ligand binding domain. The vertebrate RXR ligand binding domain may be a human RXR ligand binding domain. In specific cases, the ligand is a diacylhydrazine.

The manipulated fibroblasts may be detected in the individual in some aspects. In specific cases, the manipulated fibroblasts are detected in the mammal a certain number of hours after the fibroblasts and ligand are administered to the mammal, such as 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or more hours, for example. In particular aspects, a ligand is administered to a mammal daily for a period of about 1, 2, 3, 4, 5, 6, 7, 8, 9, 10, 11, 12, 13, 14, 15, 16, 17, 18, 19, 20, or more days. The ligand may or may not be administered to the mammal orally.

In one particular aspect of the gene switch method, a mammal is a human and the modified fibroblasts are allogeneic fibroblasts, wherein the vector is an adenoviral vector, and the polynucleotide encoding a gene switch comprises (1) a first transcription factor sequence encoding a VP-16 transactivation domain and a chimeric RXR ligand binding domain, (2) an EMCV IRES, and (3) a second transcription factor sequence encoding a GAL4 DNA-binding domain and a Choristoneura fumiferana ligand binding domain comprising a substitution mutation, wherein the ligand is a diacylhydrazine that is administered once daily for 14 consecutive days beginning on the day that the engineered fibroblasts are administered. In a specific aspect, the ligand is RG-115932.

In some aspects, immune activation is associated with fibroblast-administered therapy. In specific aspects, activated T cells are utilized together with fibroblasts that may or may not be modified. In some cases, the fibroblasts house a recombinant vector that expressed a therapeutic gene product (protein, RNA, etc.). In specific cases the fibroblasts are infected with an oncolytic virus.

Optimal T cell activation requires simultaneous signals through the T cell receptor and costimulatory molecules. The costimulatory molecule CD28, upon interaction with its ligands B7-1 and B7-2, plays a role in initial T cell priming. However, the CD28-mediated T cell expansion is opposed by the B7-1/2 counter receptor, cytotoxic T lymphocyte associated antigen 4 (CTLA-4), which mitigates the proliferation of recently activated T cells. This sequential regulation of CD28 and CTLA-4 expression balances the activating and inhibitory signals and ensures the induction of an effective immune response, while protecting against the development of autoimmunity. Blocking of CTLA-4 with monoclonal antibodies has demonstrated some success in human clinical trials. Additional CD28 and B7 family members have been identified: PD-1 (programmed death-1), PD-L1 (programmed death ligand-1 or B7-H1), and PD-L2 (B7-DC). As in the CTLA-4B7 system, the PD-1 interactions with PD-L1 and PD-L2 suppress both central and peripheral immune responses, and therefore, the PD-1 blockade is also being explored in clinical trials. In addition, numerous new agents targeting the inhibitory and activation pathways involved in T-cell modulation such as LAG-3, B7-H3, CD40, OX40, CD137 and others may be utilized.

Accordingly, in some aspects, T-cell induction comprises administration of an agonist of an activating co-stimulatory molecule. In some aspects, the method comprises administration of one or more agonistic antibodies directed against activating co-stimulatory molecules. In some aspects, T-cell induction comprises administration of one or more agonistic antibodies against a co-stimulatory molecule selected from the group consisting of CD28, OX40, GITR, CD137, CD27 and HVEM.

In some aspects, T-cell induction comprises administration of a treatment that antagonizes negative co-stimulatory molecules. In some aspects, the method comprises administration of blocking antibodies against negative co-stimulatory molecules. In some aspects, T-cell induction comprises administration of blocking antibodies against a negative co-stimulatory molecule selected from the group consisting of CTLA-1; PD-1, TIM-3, BTLA, VISTA and LAG-3. In some aspects, T-cell induction comprises administration of one or more CTLA-4 blocking antibodies. In some aspects, T-cell induction comprises administration of one or more PD-1 pathway inhibitors. In some aspects, the inhibitor of the PD-1 pathway is selected from antibodies against PD-1 and soluble PD-1 ligand. In some aspects, the inhibitors of the PD-1 pathway are selected from the group consisting of AMP-244, MEDI-4736, MPDL328 OA, MIH1, and a combination thereof.

In some aspects, T-cell induction comprises administration of one or more treatments that stimulate T-cell expansion. In some aspects, a treatment that stimulates T-cell expansion comprises administration of one or more cytokines. In some aspects, a treatment that stimulates T-cell expansion comprises administration of cytokine(s)-expressing fibroblasts.

### IV.General Aspects

Methods and compositions related to preparation and/or use of fibroblasts cells as delivery agents may or may not have general elements as follows.

The amount of any types of cells for administration to an individual may depend on the type of disease to be treated, of the severity and stage of the disease, and/or of the type of cells to be injected for the treatment. The cells may be prepared for administration in a pharmaceutically acceptable carrier, for example a sterile saline isotonic solution. In some aspects, the pharmaceutically acceptable carrier may comprise one or more additional agents, such as FAS ligand, IL-2R, IL-1 Ra, IL-2, IL-4, IL-8, IL-10, IL-20, IL-35, HLA-G, PD-L1, I-309, IDO, iNOS, CD200, Galectin 3, sCR1, arginase, PGE-2, aspirin, atorvastatin, fluvastatin, lovastatin, pravastatin, rosuvastatin, simvastatin, pitavastatin, n-acetylcysteine, rapamycin, IVIG, naltrexone, TGF-beta, VEGF, PDGF, CTLA-4, anti-CD45RB antibody, hydroxychloroquine, leflunomide, auranofin, dicyanogold, sulfasalazine, methotrexate, glucocorticoids, etanercept, adalimumab, abatacept, anakinra, certolizumab, Etanercept-szzs, golimumab, infliximab, rituximab, tocilizumab, cyclosporine, IFN-gamma, everolimus, rapamycin, VEGF, FGF-1, FGF-2, angiopoietin, HIF-1-alpha, or a combination thereof.

In one aspect of the disclosure, fibroblasts are administered to a subject by any suitable route, including by injection, including in hypoxic areas. In other aspects, given the ability of fibroblasts to home to cancer cells, the modified fibroblast cells may be provided systemically to an individual. Suitable routes include intramuscular, intravenous, subcutaneous, intrathecal, oral, intrarectal, intrathecal, intra-omental, intraventricular, intrahepatic, topical, and intrarenal.

In certain aspects, fibroblasts may be derived from tissues comprising skin, heart, blood vessels, bone marrow, skeletal muscle, liver, pancreas, brain, adipose tissue, foreskin, placental, and/or umbilical cord, for example. In specific aspects, the fibroblasts are placental, fetal, neonatal or adult or mixtures thereof.

The number of administrations of cells to an individual will depend upon the factors described herein at least in part and may be optimized using routine methods in the art. In specific aspects, a single administration is required. In other aspects, a plurality of administration of cells is required. It should be appreciated that the system is subject to variables, such as the particular need of the individual, which may vary with time and circumstances, the rate of loss of the cellular activity as a result of loss of cells or activity of individual cells, and the like. Therefore, it is expected that each individual could be monitored for the proper dosage, and such practices of monitoring an individual are routine in the art.

In some aspects, the cells are subjected to one or more media compositions that comprises, consists of, or consists essentially of Roswell Park Memorial Institute (RPMI-1640), Dublecco's Modified Essential Media (DMEM), Eagle's Modified Essential Media (EMEM), Optimem, Iscove's Media, or a combination thereof.

In one aspect of the disclosure, the fibroblast cells are cultured *ex vivo* using means known in the art for preserving viability and proliferative ability of the cells. In specific aspects for fibroblasts, there may be modification of known culture techniques to achieve one or more desired effects for the cells, such as to enhance homing capabilities, decrease visibility of fibroblasts to a recipient immune system, and so forth. In one aspect, fibroblast cells are cultured in conditions that lack one or more xenogeneic components, such as fetal calf serum. In specific aspects, the disclosure encompasses the substitution of fetal calf serum with human platelet rich plasma, platelet lysate, umbilical cord blood serum, autologous serum, and/or defined cytokine mixes as an additional feature, for example to reduce the immunogenicity of the fibroblast cells.

In some aspects, fibroblasts are frozen and/or obtained from storage before use. In one example, protocols for freezing are used as described in the art, for example, freezing solutions include 5% DMSO, 30% FBS in alpha-MEM medium (Gibco-BRL, other solutions include human albumin 4.5% solution (ZENALB 4.5, Bio Products Laboratory) containing either no DMSO (D0) or increasing concentrations of DMSO (CryoSure-DMSO, WAK-Chemie Medical) from 0.5% to 20% (D0.5-D20, respectively). Prior to freezing, cells are harvested and washed with 1 × phosphate-buffered saline; then, cell pellets directly resuspended in 1 mL of freezing solution at concentrations of 1 × 10⁶ cells/mL, 5 × 10⁶ cells/mL or 10 × 10⁶ cells/mL, transferred into cryovials followed by placing the cryovials in an isopropanol freezing box (Nalgene cryo 1°C/min freezing container, Nalgene) for overnight freezing in a -80°C freezer (New Brunswick Scientific), and then stored in liquid nitrogen vapor (Taylor-Wharton) for at least 1 week and up to 3 weeks. Before use, the cells may be thawed by rapidly immersing the cryovials in a 37°C water bath with gentle shaking for 2 min, followed by transfering cells into 9 mL of warmed α-MEM for wash and cells pelleted by centrifugation at 1100 rpm for 5 min. The pelleted cells are then assessed for viability before administration. In some aspects, migration ability and/or functionality of cells is tested.

In cases wherein recombination technology is employed, one or more types of the fibroblast cells are manipulated to harbor one or more expression vectors that each encode one or more gene products of interest. A recombinant expression vector(s) can be introduced as one or more DNA molecules or constructs, where there may be at least one marker that will allow for selection of host cells that contain the vector(s). The vector(s) can be prepared in conventional ways, wherein the genes and regulatory regions may be isolated, as appropriate, ligated, cloned in an appropriate cloning host, and analyzed by sequencing or other convenient means. Particularly, using PCR, individual fragments including all or portions of a functional unit may be isolated, where in some cases one or more mutations may be introduced using "primer repair", ligation, *in vitro* mutagenesis, *etc.* as appropriate. The vector(s) once completed and demonstrated to have the appropriate sequences may then be introduced into the host cell by any convenient means. The constructs may be integrated and packaged into non-replicating, defective viral genomes like lentivirus, Adenovirus, Adeno-associated virus (AAV), Herpes simplex virus (HSV), or others, including retroviral vectors, for infection or transduction into cells. The vector(s) may include viral sequences for transfection, if desired. Alternatively, the construct may be introduced by fusion, electroporation, biolistics, transfection, lipofection, or the like. The host cells may be grown and expanded in culture before introduction of the vector(s), followed by the appropriate treatment for introduction of the vector(s) and integration of the vector(s). The cells are then expanded and screened by virtue of a marker present in the construct. Various markers that may be used successfully include hprt, neomycin resistance, thymidine kinase, hygromycin resistance, *etc.*

Any of the genes or gene products described herein, or active portions thereof, may be cloned into mammalian expression constructs comprising one or more promoter sequences enabling expression in cells such as the CMV promoter [Artuc et al., Exp. Dermatol. 1995, 4:317-21]. Examples of suitable constructs include, but are not limited to pcDNA3, pcDNA3.1 (+/-), pGL3, PzeoSV2 (+/-), pDisplay, pEF/myc/cyto, pCMV/myc/cyto each of which is commercially available, or the pSH expression vector which enables a regulated polynucleotide expression in human foreskin cells [Ventura and Villa, 1993, Biochem. Biophys. Commun. 192: 867-9]. Examples of retroviral vector and packaging systems are those sold by Clontech, San Diego, Calif., USA, including Retro-X vectors pLNCX and pLXSN, which permit cloning into multiple cloning sites and the transgene is transcribed from CMV promoter. Vectors derived from Mo-MuLV are also included such as pBabe, where the transgene will be transcribed from the 5'LTR promoter. After completing plasmid transfection fibroblasts are harvested by a means allowing for detachment from tissue culture plates, for example, by trypsinization and transferred to either a 6-well (Nunc, Denmark) or a 24-well plate (Nunc) for proliferation. Approximately 3 days post-transfection, the cell media is changed to media allow for proliferation and expansion of modified fibroblasts. One example is Neurobasal A (NBA) proliferation medium comprising Neurobasal-A (Invitrogen), 1% D-glucose (Sigma Aldrich), 1% Penicillin/Streptomycin/Glutamine (Invitrogen), 2% B27 supplement with Retinoic acid (Invitrogen), 0.2% EGF (Peprotech, USA), 0.08% FGF-2 (Peprotech), 0.2% Heparin (Sigma Aldrich, USA) and Valproic acid (Sigma-Aldrich) to a concentration of 1 µM. The media is then subsequently changed thrice weekly, and cells are re-plated regularly (for example, 2-8 times up to a maximum of weekly re-plating, becoming more regular as colonies began to develop) to remove non-reprogrammed cells until widespread colony formation is achieved.

In some instances, one or more agents may be introduced into the cells as an RNA molecule for transient expression. RNA can be delivered to any cells, including any modified cells, of the disclosure by various means including microinjection, electroporation, and lipid-mediated transfection, for example. In particular aspects, introduction of vector(s) into cells may occur *via* transposons. An example of a synthetic transposon for use is the Sleeping Beauty transposon that comprises an expression cassette including the angiogenic agent gene thereof. Alternatively, one may have a target site for homologous recombination, where it is desired that vector(s) be integrated at a particular locus using materials and methods as are known in the art for homologous recombination. For homologous recombination, one may use either OMEGA or O-vectors. See, for example, Thomas and Capecchi, 1987; Mansour, *et al.,* 1988; and Joyner, *et al.,* 1989.

The vector(s) may be introduced as a single DNA molecule encoding at least one agent (including one or more tumor inhibitory agents or functional fragments thereof) and optionally another polynucleotide (such as genes), or different DNA molecules having one or more polynucleotides (such as genes). The vector(s) may be introduced simultaneously or consecutively, each with the same or different markers. In an illustrative example, one vector would contain one or more agents (such as angiogenic agent(s)) under the control of particular regulatory sequences.

Vector(s) comprising useful elements such as bacterial or yeast origins of replication, selectable and/or amplifiable markers, promoter/enhancer elements for expression in prokaryotes or eukaryotes, *etc.* that may be used to prepare stocks of vector DNAs and for carrying out transfections are well known in the art, and many are commercially available.

In certain aspects, it is contemplated that RNAs or proteinaceous sequences may be co-expressed with other selected RNAs or proteinaceous sequences in the same host cell. Co-expression may be achieved by co-transfecting the host cell with two or more distinct recombinant vectors. Alternatively, a single recombinant vector may be constructed to include multiple distinct coding regions for RNAs, which could then be expressed in host cells transfected with the single vector.

In some situations, it may be desirable to kill the modified cells, such as when the object is to terminate the treatment, the cells become neoplastic, in research where the absence of the cells after their presence is of interest, and/or another event. For this purpose one can provide for the expression of certain gene products in which one can kill the modified cells under controlled conditions, such as a suicide gene. Suicide genes are known in the art, *e.g.* the iCaspase9 system in which a modified form of caspase 9 is dimerizable with a small molecule, *e.g.* AP1903. See, *e.g.,* Straathof et al., Blood 105:4247-4254 (2005).

### V. Kits of the Disclosure

Any of the cellular and/or non-cellular compositions described herein or similar thereto may be comprised in a kit. In a non-limiting example, one or more reagents for use in methods for preparing fibroblasts may be comprised in a kit. Such reagents may include cells, vectors, one or more growth factors, vector(s) one or more costimulatory factors, media, enzymes, buffers, nucleotides, salts, primers, and so forth. The kit components are provided in suitable container means.

Some components of the kits may be packaged either in aqueous media or in lyophilized form. The container means of the kits will generally include at least one vial, test tube, flask, bottle, syringe or other container means, into which a component may be placed, and preferably, suitably aliquoted. Where there are more than one component in the kit, the kit also will generally contain a second, third or other additional container into which the additional components may be separately placed. However, various combinations of components may be comprised in a vial. The kits of the present disclosure also will typically include a means for containing the components in close confinement for commercial sale. Such containers may include injection or blow molded plastic containers into which the desired vials are retained.

When the components of the kit are provided in one and/or more liquid solutions, the liquid solution is an aqueous solution, with a sterile aqueous solution being particularly useful. In some cases, the container means may itself be a syringe, pipette, and/or other such like apparatus, or may be a substrate with multiple compartments for a desired reaction.

Some components of the kit may be provided as dried powder(s). When reagents and/or components are provided as a dry powder, the powder can be reconstituted by the addition of a suitable solvent. It is envisioned that the solvent may also be provided in another container means. The kits may also comprise a second container means for containing a sterile acceptable buffer and/or other diluent.

In specific aspects, reagents and materials include primers for amplifying desired sequences, nucleotides, suitable buffers or buffer reagents, salt, and so forth, and in some cases the reagents include apparatus or reagents for isolation of a particular desired cell(s).

In particular aspects, there are one or more apparatuses in the kit suitable for extracting one or more samples from an individual. The apparatus may be a syringe, fine needles, scalpel, and so forth.

### EXAMPLES

The following examples are included to demonstrate preferred aspects of the disclosure.

### EXAMPLE 1

### PRODUCTION OF FIBROBLASTS AS ANTI-TUMOR DELIVERY AGENTS

In one particular aspect, fibroblasts are treated to increase expression of receptors involved in homing to cancers and in a specific aspect they are treated with hypoxia. In one specific aspect, receptors involved in homing to cancers comprise one or more chemokine receptors. Specific chemokine receptors include CXCR4 receptor. Fibroblasts possessing enhanced tumor migratory activity may be subsequently infected with an immune stimulatory virus, in specific aspects, such as an oncolytic virus including vaccinia virus. In some aspects, increased susceptibility to infection is achieved by culture with histone deacetylase inhibitors, such as valporic acid. Subsequent to administration of cells that are virally infected, administration of chemotherapy (including that capable of stimulating immunity) is performed. The chemotherapy may include low dose cyclophosphamide, including administered by various regimens such as metronomic administration in order to decrease T regulatory cells and enhance antitumor immunity [53-67]. Other agents alone or together with cyclophosphamide may be used, such as gemcitabine [68], everolimus [69], and doxorubicin [70], or a combination thereof.

### EXAMPLE 2

### FIBROBLAST DELIVERY OF TUMOR INHIBITORY AGENTS

C57 BL/6 fibroblasts where obtained from dermal samples, dissociated using collagenase, and cultured in RPMI 1640 media supplemented with 10% fetal calf serum. Cells where infected with 100,000 plaque forming units (PFU) of Newcastle Disease Virus. Subsequent to a 24 hour culture to allow for infection, fibroblasts where harvested by trypsinization and utilized for treatment of cancer bearing mice. Fibroblasts where administered at day 18 after tumor administration.

B16 murine melanoma cells were maintained in Iscove's IMDM (BioWhittaker) supplemented with 1 U/ml penicillin, 1 µg/ml streptomycin, 2 µM 1-glutamine, 20 µM β-mercaptoethanol (complete medium), and 10% fetal calf serum.

Tumors were induced by subcutaneously administering the right flank with 2.5 × 10³ (or the amount indicated) B16-BL6 tumor cells in 200 µl PBS-0.1% BSA.

Female C57B6 Mice (10 per group) were treated with saline (diamond), uninfected fibroblasts, 100,000 cells administered intravenously, (square), or Newcastle disease virus infected fibroblasts (triangle) until day 31 .

Tumor growth was assessed every 3 days by two measurements of perpendicular diameters by a caliper, and animals were sacrificed when tumors reached a size of 1 cm in any direction. Tumor volume was calculated by the following formula: (the shortest diameter2 × the longest diameter)/2.

As observed in FIG. 1, significant inhibition of growing tumor was observed in mice administered fibroblasts transfected with Newcastle disease virus. Administration of Newcastle disease virus alone, even at concentrations as high as 200000 PFU per mouse did not affect tumor growth (data not shown).

### REFERENCES

All publications mentioned in the specification are indicative of the level of those skilled in the art to which the invention pertains.
1. Kucerova, L., et al., Targeted antitumor therapy mediated by prodrug-activating mesenchymal stromal cells. Cancer Lett, 2017.
2. Tehrani, R.M., et al., Mesenchymal Stem Cells: A New Platform for Targeting Suicide Genes in Cancer. J Cell Physiol, 2017.
3. Song, C., et al., Thymidine kinase gene modified bone marrow mesenchymal stem cells as vehicles for antitumor therapy. Hum Gene Ther, 2011. 22(4): p. 439-49.
4. Yang, Y., et al., Targeting eradication of malignant cells derived from human bone marrow mesenchymal stromal cells. Exp Cell Res, 2010. 316(20): p. 3329-41.
5. Miletic, H., et al., Bystander killing of malignant glioma by bone marrow-derived tumor-infiltrating progenitor cells expressing a suicide gene. Mol Ther, 2007. 15(7): p. 1373-81.
6. Cavarretta, I.T., et al., Adipose tissue-derived mesenchymal stem cells expressing prodrug-converting enzyme inhibit human prostate tumor growth. Mol Ther, 2010. 18(1): p. 223-31.
7. Kucerova, L., et al., Cytosine deaminase expressing human mesenchymal stem cells mediated tumour regression in melanoma bearing mice. J Gene Med, 2008. 10(10): p. 1071-82.
8. Toro, L., et al., Metastatic Ovarian Cancer Can Be Efficiently Treated by Genetically Modified Mesenchymal Stromal Cells. Stem Cells Dev, 2016.
9. Moreno, R., et al., Human Menstrual Blood DerivedMesenchymal Stem Cells as Potential Cell Carriers for Oncolytic Adenovirus. Stem Cells Int, 2017. 2017: p. 3615729.
10. Hakkarainen, T., et al., Human mesenchymal stem cells lack tumor tropism but enhance the antitumor activity of oncolytic adenoviruses in orthotopic lung and breast tumors. Hum Gene Ther, 2007. 18(7): p. 627-41.
11. Komarova, S., et al., Mesenchymal progenitor cells as cellular vehicles for delivery of oncolytic adenoviruses. Mol Cancer Ther, 2006. 5(3): p. 755-66.
12. Li, Y., et al., Anti-cancer effect of oncolytic adenovirus-armed shRNA targeting MYCN gene on doxorubicin-resistant neuroblastoma cells. Biochem Biophys Res Commun, 2017. 491(1): p. 134-139.
13. Niemann, J. and F. Kuhnel, Oncolytic viruses: adenoviruses. Virus Genes, 2017.
14. Tazawa, H., et al., Impact of Autophagy in Oncolytic Adenoviral Therapy for Cancer. Int J Mol Sci, 2017. 18(7).
15. Futami, M., et al., Efficacy and Safety of Doubly-Regulated Vaccinia Virus in a Mouse Xenograft Model of Multiple Myeloma. Mol Ther Oncolytics, 2017. 6: p. 57-68.
16. Esaki, S., et al., Blockade of transforming growth factor-beta signaling enhances oncolytic herpes simplex virus efficacy in patient-derived recurrent glioblastoma models. Int J Cancer, 2017.
17. Liu, X.J., et al., [Oncolytic property of HSV-1 recombinant viruses carrying the human IL-12J. Zhonghua Yi Xue Za Zhi, 2017. 97(27): p. 2135-2140.
18. Du, W., et al., Stem cell-released oncolytic herpes simplex virus has therapeutic efficacy in brain metastatic melanomas. Proc Natl Acad Sci USA, 2017. 114(30): p. E6157-E6165.
19. Moesta, A.K., et al., Local Delivery of OncoVEXmGM-CSF Generates Systemic Anti-Tumor Immune Responses Enhanced by Cytotoxic T Lymphocyte Associated Protein Blockade. Clin Cancer Res, 2017.
20. Yin, J., J.M. Markert, and J.W. Leavenworth, Modulation of the Intratumoral Immune Landscape by Oncolytic Herpes Simplex Virus Virotherapy. Front Oncol, 2017. 7: p. 136.
21. Cohn, D.E., et al., Randomized phase IIB evaluation of weekly paclitaxel versus weekly paclitaxel with oncolytic reovirus (Reolysin(R)) in recurrent ovarian, tubal, or peritoneal cancer: An NRG Oncology/Gynecologic Oncology Group study. Gynecol Oncol, 2017. 146(3): p. 477-483.
22. Chen, A., et al., Oncolytic measles virus enhances antitumour responses of adoptive CD8+NKG2D+ cells in hepatocellular carcinoma treatment. Sci Rep, 2017. 7(1): p. 5170.
23. Kleinlutzum, D., et al., Enhancing the Oncolytic Activity of CD733-Targeted Measles Virus: Receptor Extension or Chimerism with Vesicular Stomatitis Virus Are Most Effective. Front Oncol, 2017. 7: p. 127.
24. Li, Q., et al., alpha2,6-linked sialic acid serves as a high-affinity receptor for cancer oncolytic virotherapy with Newcastle disease virus. J Cancer Res Clin Oncol, 2017.
25. Rangaswamy, U.S., et al., Newcastle Disease Virus Establishes Persistent Infection in Tumor Cells In Vitro: Contribution of the Cleavage Site of Fusion Protein and Second Sialic Acid Binding Site of Hemagglutinin Neuraminidase. J Virol, 2017. 91(16).
26. Liao, Y., et al., RIP1 is a central signaling protein in regulation of TNF-alpha/TRAIL mediated apoptosis and necroptosis during Newcastle disease virus infection. Oncotarget, 2017. 8(26): p. 43201-43217.
27. Loebinger, M.R., et al., Mesenchymal stem cell delivery of TRAIL can eliminate metastatic cancer. Cancer Res, 2009. 69(10): p. 4134-42.
28. Xia, L., et al., TRAIL-expressing gingival-derived mesenchymal stem cells inhibit tumorigenesis of tongue squamous cell carcinoma. J Dent Res, 2015. 94(1): p. 219-28.
29. Attar, R., et al., TRAIL based therapy: overview of mesenchymal stem cell based delivery and miRNA controlled expression of TRAIL. Asian Pac J Cancer Prev, 2014. 15(16): p. 6495-7.
30. Loebinger, M.R., et al., TRAIL-expressing mesenchymal stem cells kill the putative cancer stem cell population. Br J Cancer, 2010. 103(11): p. 1692-7.
31. Yuan, Z., et al., Mesenchymal stromal cell delivery of full-length tumor necrosis factor-related apoptosis-inducing ligand is superior to soluble type for cancer therapy. Cytotherapy, 2015. 17(7): p. 885-96.
32. Choi, S.A., et al., Preclinical Biosafety Evaluation of Genetically Modified Human Adipose Tissue-Derived Mesenchymal Stem Cells for Clinical Applications to Brainstem Glioma. Stem Cells Dev, 2016. 25(12): p. 897-908.
33. Yan, C., et al., Human umbilical cord mesenchymal stem cells delivering sTRAIL home to lung cancer mediated by MCP-1/CCR2 axis and exhibit antitumor effects. Tumour Biol, 2016. 37(6): p. 8425-35.
34. Grisendi, G., et al., Mesenchymal progenitors expressing TRAIL induce apoptosis in sarcomas. Stem Cells, 2015. 33(3): p. 859-69.
35. Zhang, B., et al., The inhibitory effect of MSCs expressing TRAIL as a cellular delivery vehicle in combination with cisplatin on hepatocellular carcinoma. Cancer Biol Ther, 2012. 13(12): p. 1175-84.
36. Ciavarella, S., et al., In vitro anti-myeloma activity of TRAIL-expressing adipose-derived mesenchymal stem cells. Br J Haematol, 2012. 157(5): p. 586-98.
37. Zhang, X., et al., Mesenchymal stem cells modified to express lentivirus TNF-alpha Tumstatin(45-132) inhibit the growth of prostate cancer. J Cell Mol Med, 2011. 15(2): p. 433-44.
38. Yang, X., et al., IFN-gamma-secreting-mesenchymal stem cells exert an antitumor effect in vivo via the TRAIL pathway. J Immunol Res, 2014. 2014: p. 318098.
39. Su, Y., et al., Interferon-alpha2b gene-modified human bone marrow mesenchymal stem cells inhibit hepatocellular carcinoma by reducing the Notch1 levels. Life Sci, 2015. 143: p. 18-26.
40. Xie, C., et al., Interferon-beta gene-modified human bone marrow mesenchymal stem cells attenuate hepatocellular carcinoma through inhibiting AKT/FOXO3a pathway. Br J Cancer, 2013. 109(5): p. 1198-205.
41. Dembinski, J.L., et al., Tumor stroma engraftment of gene-modified mesenchymal stem cells as anti-tumor therapy against ovarian cancer. Cytotherapy, 2013. 15(1): p. 20-32.
42. Wang, G.X., et al., Mesenchymal stem cells modified to express interferon-beta inhibit the growth of prostate cancer in a mouse model. J Int Med Res, 2012. 40(1): p. 317-27.
43. Ren, C., et al., Cancer gene therapy using mesenchymal stem cells expressing interferon-beta in a mouse prostate cancer lung metastasis model. Gene Ther, 2008. 15(21): p. 1446-53.
44. Ryu, C.H., et al., Gene therapy of intracranial glioma using interleukin 12-secreting human umbilical cord blood-derived mesenchymal stem cells. Hum Gene Ther, 2011. 22(6): p. 733-43.
45. Seo, S.H., et al., The effects of mesenchymal stem cells injected via different routes on modified IL-12-mediated antitumor activity. Gene Ther, 2011. 18(5): p. 488-95.
46. Liu, X., et al., Mesenchymal stem cells expressing interleukin-18 suppress breast cancer cells in vitro. Exp Ther Med, 2015. 9(4): p. 1192-1200.
47. Sun, S., et al., [Effect of interleukin-18 gene modified human umbilical cord mesenchymal stem cells on proliferation of breast cancer cell]. Zhonghua Yi Xue Za Zhi, 2014. 94(26): p. 2013-7.
48. Kim, N., et al., IL-21-Expressing Mesenchymal Stem Cells Prevent Lethal B-Cell Lymphoma Through Efficient Delivery of IL-21, Which Redirects the Immune System to Target the Tumor. Stem Cells Dev, 2015. 24(23): p. 2808-21.
49. Suzuki, T., et al., Mesenchymal stem cells are efficiently transduced with adenoviruses bearing type 35-derived fibers and the transduced cells with the IL-28A gene produces cytotoxicity to lung carcinoma cells co-cultured. BMC Cancer, 2014. 14: p. 713.
50. Aliperta, R., et al., Cryogel-supported stem cell factory for customized sustained release of bispecific antibodies for cancer immunotherapy. Sci Rep, 2017. 7: p. 42855.
51. Cai, Y., et al., Dual targeting and enhanced cytotoxicity to HER2-overexpressing tumors by immunoapoptotin-armored mesenchymal stem cells. Cancer Lett, 2016. 381(1): p. 104-12.
52. Csatary, L.K., et al., Attenuated veterinary virus vaccine for the treatment of cancer. Cancer Detect Prev, 1993. 17(6): p. 619-27.
53. Loyher, P.L., et al., CCR2 Influences T Regulatory Cell Migration to Tumors and Serves as a Biomarker of Cyclophosphamide Sensitivity. Cancer Res, 2016. 76(22): p. 6483-6494.
54. Ahlmann, M. and G. Hempel, The effect of cyclophosphamide on the immune system: implications for clinical cancer therapy. Cancer Chemother Pharmacol, 2016. 78(4): p. 661-71.
55. Abu Eid, R., et al., Old School Chemotherapy in Immunotherapeutic Combination in Cancer, A Low-cost Drug Repurposed. Cancer Immunol Res, 2016. 4(5): p. 377-82.
56. Noguchi, M., et al., A randomized phase II clinical trial of personalized peptide vaccination with metronomic low-dose cyclophosphamide in patients with metastatic castration-resistant prostate cancer. Cancer Immunol Immunother, 2016. 65(2): p. 151-60.
57. Klein, O., et al., Low-dose cyclophosphamide enhances antigen-specific CD4(+) T cell responses to NY-ESO-1/ISCOMATRIX vaccine in patients with advanced melanoma. Cancer Immunol Immunother, 2015. 64(4): p. 507-18.
58. Weir, G.M., et al., Metronomic cyclophosphamide enhances HPV16E7 peptide vaccine induced antigen-specific and cytotoxic T-cell mediated antitumor immune response. Oncoimmunology, 2014. 3(8): p. e953407.
59. Peng, S., et al., Low-dose cyclophosphamide administered as daily or single dose enhances the antitumor effects of a therapeutic HPV vaccine. Cancer Immunol Immunother, 2013. 62(1): p. 171-82.
60. Ge, Y., et al., Metronomic cyclophosphamide treatment in metastasized breast cancer patients: immunological effects and clinical outcome. Cancer Immunol Immunother, 2012. 61(3): p. 353-62.
61. Mkrtichyan, M., et al., Anti-PD-1 synergizes with cyclophosphamide to induce potent anti-tumor vaccine effects through novel mechanisms. Eur J Immunol, 2011. 41(10): p. 2977-86.
62. Maglioco, A., et al., Lymphadenectomy exacerbates tumor growth while lymphadenectomy plus the adoptive transfer of autologous cytotoxic cells and low-dose cyclophosphamide induces regression of an established murine fibrosarcoma. Cancer Immunol Immunother, 2011. 60(3): p. 389-99.
63. Lasalvia-Prisco, E., et al., Randomized phase II clinical trial of chemo-immunotherapy in advanced nonsmall cell lung cancer. Biologics, 2008. 2(3): p. 555-61.
64. Leao, I.C., et al., Effective depletion of regulatory T cells allows the recruitment of mesothelin-specific CD8 T cells to the antitumor immune response against a mesothelin-expressing mouse pancreatic adenocarcinoma. Clin Transl Sci, 2008. 1(3): p. 228-39.
65. Lord, R., et al., Low dose metronomic oral cyclophosphamide for hormone resistant prostate cancer: a phase II study. J Urol, 2007. 177(6): p. 2136-40; discussion 2140.
66. Liu, J.Y., et al., Single administration of low dose cyclophosphamide augments the antitumor effect of dendritic cell vaccine. Cancer Immunol Immunother, 2007. 56(10): p. 1597-604.
67. Loeffler, M., J.A. Kruger, and R.A. Reisfeld, Immunostimulatory effects of low-dose cyclophosphamide are controlled by inducible nitric oxide synthase. Cancer Res, 2005. 65(12): p. 5027-30.
68. Tongu, M., et al., Metronomic chemotherapy with low-dose cyclophosphamide plus gemcitabine can induce anti-tumor T cell immunity in vivo. Cancer Immunol Immunother, 2013. 62(2): p. 383-91.
69. Huijts, C.M., et al., Phase I-II study of everolimus and low-dose oral cyclophosphamide in patients with metastatic renal cell cancer. BMC Cancer, 2011. 11: p. 505.
70. Tongu, M., et al., Immunogenic chemotherapy with cyclophosphamide and doxorubicin against established murine carcinoma. Cancer Immunol Immunother, 2010. 59(5): p. 769-77.

## Claims

1. An *in vitro* method of preparing fibroblasts as anti-cancer delivery compositions, comprising the steps of:
(a) modifying fibroblasts to enhance activity of homing to cancer cells or tissue comprising cancer cells; and
(b) manipulating fibroblasts to comprise one or more tumor inhibitory agents,
wherein the fibroblasts of step (a), step (b), or both are exposed to hypoxia under suitable conditions and wherein the tumor inhibitory agent comprises a vector that expresses interleukin-12 (IL-12).

2. The method of claim 1, wherein:
(i) step (a) occurs before step (b); or
(ii) step (a) occurs after step (b); or
(iii) step (a) and step (b) occur concomitantly.

3. The method of claim 1 or 2, wherein modifying fibroblasts to enhance activity of homing to cancer cells or tissue comprising cancer cells comprises transfecting the fibroblasts with one or more chemokine receptors, optionally wherein the chemokine receptor is chemokine(C-X-C) receptor 4 (CXCR4) receptor, CXCL16 receptor and/or CCL9 receptor.

4. The method of any one of claims 1-3, wherein:
(a) the hypoxia is from 0.1%-10%, 0.1%-5%, 0.1%-2.5%, 0.1%-2%, 0.1%-1%, 0.5%-10%, 0.5%-7.5%, 0.5%-5%, 0.5%-2.5%, 0.5%-2%, 0.5%-1%, 1%-10%, 1%-7.5%, 1%-5%, 1%-2.5%, 1%-2%, 2%-10%, 2%-7.5%, 2%-5%, 2%-2.5%, 5%-10%, 5%-7.5%, 5%-6%, or 7.5%-10% oxygen; and/or
(b) the hypoxia is exposed to the fibroblasts for a duration of 30 minutes (min)-3 days, 30 min-2 days, 30 min-1 day, 30 min-12 hours (hrs), 30 min-8 hrs, 30 min-6 hrs, 30 min-4 hrs, 30 min-2 hrs, 30 min-1 hr, 1 hr-3 days, 1hr-2 days, 1 hr-1 day, 1-12 hrs, 1-8 hrs, 1-6 hrs, 1-4 hrs, 1-2 hrs, 2 hrs-3 days, 2hrs-2 days, 2 hrs-1 day, 2 hrs-12 hrs, 2-10hrs, 2-8hrs, 2-6 hrs, 2-4 hrs, 2-3 hrs, 6 hrs-3 days, 6 hrs-2 days, 6 hrs-1 day, 6-12 hrs, 6-8 hrs, 8hrs-3 days, 8 hrs-2 days, 8 hrs-1 day, 8-16 hrs, 8-12 hrs, 8-10 hrs, 12hrs-3 days, 12 hrs-2 days, 12 hrs-1 day, 12-18hrs, 12-14hrs, 1-3 days, or 1-2 days.

5. The method of any one of claims 1-4, wherein the tumor inhibitory agent is a nucleic acid, protein, or peptide.

6. The method of any one of claims 1-4, wherein the tumor inhibitory agent is a virus, optionally
wherein the virus is an oncolytic virus or tumor trophic virus, optionally
wherein the oncolytic virus is vaccinia virus.

7. The method of any one of claims 1-6, wherein the vector further expresses TNF-alpha, TNF-related apoptosis-inducing ligand (TRAIL), a suicide gene, thymidylate synthase, bone morphogenic protein 4 (BMP4), HSV-thymidine kinase, an oncolytic adenovirus, interleukin-2 (IL-2), interleukin-18 (IL-18), interleukin-23 (IL-23), Interferon-alpha, Interferon-beta, bispecific antibody, obestatin, XIAP, survivin, BCL-2, BCL-XL, GATA-4, IGF-1, EGF, heme-oxygenase-1, NF-kB, akt, pi3-k, epha-2, or a combination thereof.

8. The method of any one of claims 1-7, wherein the fibroblasts express one or more recombinant cytokines, optionally
wherein the recombinant cytokine is selected from interleukin (IL), colony-stimulating factor (CSF), interferon (IFN), stem cell factor (SCF), tumor growth factors (TGF), tumor necrosis factor (TNF), or a combination thereof.

9. The method of any one of claims 1-8, wherein the fibroblasts of step (a), step (b), or both are exposed or have been exposed to:
(a) one or more histone deacetylase inhibitors; or
(b) one or more immunotherapeutic molecules, optionally wherein the immunotherapeutic molecule is an antibody, optionally wherein the antibody is a bispecific antibody.

10. Fibroblasts prepared by the method of any one of claims 1-9 for use in a method of treating cancer, wherein the method comprises administering an effective amount of the modified fibroblasts to an individual.

11. The fibroblasts for use according to claim 10, wherein:
(a) the individual has cancer or is at risk for having cancer; and/or
(b) the individual is provided one or more additional anti-cancer agents, optionally
wherein the one or more additional anti-cancer agents comprises surgery, chemotherapy, radiation, hormone therapy, and/or immunotherapy.

## Patentansprüche

1. *In-vitro-*Verfahren zur Herstellung von Fibroblasten als Anti-Krebs-Abgabezusammensetzungen, das die folgenden Schritte umfasst:
(a) Modifizieren von Fibroblasten, um die Aktivität des Homing zu Krebszellen oder Gewebe, das Krebszellen umfasst, zu erhöhen; und
(b) Manipulieren von Fibroblasten, um einen oder mehrere tumorhemmende Wirkstoffe zu umfassen,
wobei die Fibroblasten von Schritt (a), Schritt (b) oder beiden unter geeigneten Bedingungen Hypoxie ausgesetzt werden und wobei das tumorhemmende Mittel einen Vektor umfasst, der Interleukin-12 (IL-12) exprimiert.

2. Verfahren nach Anspruch 1, wobei:
(i) Schritt (a) vor Schritt (b) erfolgt; oder
(ii) Schritt (a) nach Schritt (b) erfolgt; oder
(iii) Schritt (a) und Schritt (b) gleichzeitig erfolgen.

3. Verfahren nach Anspruch 1 oder 2, wobei das Modifizieren von Fibroblasten zur Verstärkung der Aktivität des Homing zu Krebszellen oder Krebszellen enthaltendem Gewebe das Transfizieren der Fibroblasten mit einem oder mehreren Chemokinrezeptoren umfasst, optional wobei der Chemokinrezeptor ein Chemokin(C-X-C)-Rezeptor 4 (CXCR4)-Rezeptor, CXCL16-Rezeptor und/oder CCL9-Rezeptor ist.

4. Verfahren nach einem der Ansprüche 1-3, wobei:
(a) die Hypoxie von 0,1%-10%, 0,1%-5%, 0,1%-2,5%, 0,1%-2%, 0,1%-1%, 0,5%-10%, 0,5%-7,5%, 0,5%-5%, 0,5%-2,5%, 0,5%-2%, 0,5%-1%, 1%-10%, 1%-7,5%, 1%-5%, 1%-2,5%, 1%-2%, 2%-10%, 2%-7,5%, 2%-5%, 2%-2,5%, 5%-10%, 5%-7,5%, 5%-6%, oder 7,5%-10% Sauerstoff reicht; und/oder
(b) die Hypoxie den Fibroblasten ausgesetzt wird für eine Dauer von 30 Minuten (min)-3 Tagen, 30 min-2 Tagen, 30 min-1 Tag, 30 min-12 Stunden (Std.), 30 min-8 Std., 30 min-6 Std., 30 min-4 Std., 30 min-2 Std., 30 min-1 Std., 1 Std.-3 Tagen, 1 Std.-2 Tagen, 1 Std.-1 Tag, 1-12 Std., 1-8 Std., 1-6 Std, 1-4 Std., 1-2 Std., 2 Std.-3 Tage, 2 Std.-2 Tage, 2 Std.-1 Tag, 2 Std.-12 Std., 2-10 Std., 2-8 Std., 2-6 Std., 2-4 Std., 2-3 Std., 6 Std.-3 Tage, 6 Std.-2 Tage, 6 Std.-1 Tag, 6-12 Std., 6-8 Std., 8 Std.-3 Tage, 8 Std.-2 Tage, 8 Std.-1 Tag, 8-16 Std., 8-12 Std., 8-10 Std., 12 Std.-3 Tage, 12 Std.-2 Tage, 12 Std.-1 Tag, 12-18 Std., 12-14 Std., 1-3 Tage, oder 1-2 Tage.

5. Verfahren nach einem der Ansprüche 1-4, wobei das tumorhemmende Mittel eine Nukleinsäure, ein Protein oder ein Peptid ist.

6. Verfahren nach einem der Ansprüche 1-4, wobei das tumorhemmende Mittel ein Virus ist, optional
wobei das Virus ein onkolytisches Virus oder ein tumortrophisches Virus ist, optional
wobei das onkolytische Virus ein Vaccinia-Virus ist.

7. Verfahren nach einem der Ansprüche 1-6, wobei der Vektor ferner Folgendes exprimiert: TNF alpha, TNF-verwandten Apoptose-induzierenden Liganden (TRAIL), ein Selbstmordgen, Thymidylat-Synthase, Knochenmorphogenes Protein 4 (BMP4), HSV-Thymidin-Kinase, ein onkolytisches Adenovirus, Interleukin-2 (IL-2), Interleukin-18 (IL-18), Interleukin-23 (IL-23), Interferon-alpha, Interferon-beta, bispezifischen Antikörper, Obestatin, XIAP, Survivin, BCL-2, BCL-XL, GATA-4, IGF-1, EGF, Häm-Oxygenase-1, NF-kB, akt, pi3-k, epha-2, oder eine Kombination davon.

8. Verfahren nach einem der Ansprüche 1-7, wobei die Fibroblasten ein oder mehrere rekombinante Zytokine exprimieren, optional
wobei das rekombinante Zytokin ausgewählt ist aus Interleukin (IL), Koloniestimulierendem Faktor (Colony Stimulating Factor, CSF), Interferon (IFN), Stammzellfaktor (Stem Cell Factor, SCF), Tumorwachstumsfaktoren (Tumor Growth Factors, TGF), Tumornekrosefaktor (TNF) oder einer Kombination davon.

9. Verfahren nach einem der Ansprüche 1-8, wobei die Fibroblasten aus Schritt (a), Schritt (b) oder beiden exponiert sind oder exponiert worden sind gegenüber:
(a) einem oder mehreren Histon-Deacetylase-Inhibitoren; oder
(b) einem oder mehreren immuntherapeutischen Molekülen, optional wobei das immuntherapeutische Molekül ein Antikörper ist, optional wobei der Antikörper ein bispezifischer Antikörper ist.

10. Fibroblasten, hergestellt durch das Verfahren nach einem der Ansprüche 1-9, zur Verwendung in einem Verfahren zur Behandlung von Krebs, wobei das Verfahren die Verabreichung einer wirksamen Menge der modifizierten Fibroblasten an ein Individuum umfasst.

11. Fibroblasten zur Verwendung nach Anspruch 10, wobei:
(a) das Individuum Krebs hat oder das Risiko hat, Krebs zu haben; und/oder
(b) dem Individuum ein oder mehrere zusätzliche Anti-Krebs-Mittel verabreicht werden, optional
wobei das eine oder die mehreren zusätzlichen Anti-Krebs-Mittel eine Operation, Chemotherapie, Bestrahlung, Hormontherapie und/oder Immuntherapie umfassen.

## Revendications

1. Procédé *in vitro* de préparation de fibroblastes comme compositions d'administration anticancéreuses, comprenant les étapes suivantes :
(a) la modification de fibroblastes pour améliorer l'activité de localisation vers des cellules cancéreuses ou des tissus comprenant des cellules cancéreuses ; et
(b) la manipulation de fibroblastes pour comprendre un ou plusieurs agents inhibiteurs de tumeur, les fibroblastes de l'étape (a), de l'étape (b) ou des deux étant exposés à l'hypoxie dans des conditions appropriées et l'agent inhibiteur de tumeur comprenant un vecteur qui exprime l'interleukine 12 (IL-12).

2. Procédé selon la revendication 1, dans lequel :
(i) l'étape (a) se produit avant l'étape (b) ; ou
(ii) l'étape (a) se produit après l'étape (b) ; ou
(iii) l'étape (a) et l'étape (b) se produisent de manière concomitante.

3. Procédé selon la revendication 1 ou 2, dans lequel la modification des fibroblastes pour améliorer l'activité de déplacement vers des cellules cancéreuses ou des tissus comprenant des cellules cancéreuses comprend la transfection des fibroblastes avec un ou plusieurs récepteurs de chimiokine, éventuellement dans lequel le récepteur de chimiokine est le récepteur 4 (CXCR4) de chimiokine (CXC), le récepteur CXCL16 et/ou le récepteur CCL9.

4. Procédé selon l'une quelconque des revendications 1 à 3, dans lequel :
(a) l'hypoxie est de 0,1 % à 10 %, 0,1 % à 5 %, 0,1 % à 2,5 %, 0,1 % à 2 %, 0,1 % à 1 %, 0,5 % à 10 %, 0,5 % à 7,5 %, 0,5 % à 5%, 0,5% à 2,5%, 0,5% à 2%, 0,5% à 1%, 1% à 10%, 1% à 7,5%, 1% à 5%, 1% à 2,5%, 1% à 2 %, 2 % à 10 %, 2 % à 7,5 %, 2 % à 5 %, 2 % à 2,5 %, 5 % à 10 %, 5 % à 7,5 %, 5 % à 6 % ou 7,5 % à 10 % d'oxygène ; et/ou
(b) l'hypoxie est exposée aux fibroblastes pendant une durée de 30 minutes (min) à 3 jours, 30 min à 2 jours, 30 min à 1 jour, 30 min à 12 heures (h), 30 min à 8 heures, 30 min à 6 h, 30 min à 4 h, 30 min à 2 h, 30 min à 1 h, 1 h à 3 jours, 1 h à 2 jours, 1 h à 1 jour, 1 à 12 h, 1 à 8 h, 1 à 6 h, 1 à4 h, 1 à2 h, 2 h à3 jours, 2 h à 2 jours, 2 h à 1 jour, 2 h à 12 h, 2 à 10 h, 2 à 8 h, 2 à 6 h, 2 à4 h, 2 à3 h, 6 h à3 jours, 6 h à 2 jours, 6 h à 1 jour, 6 à12 h, 6 à8 h, 8 h à 3 jours, 8 h à 2 jours, 8h à 1 jour, 8 à 16h, 8 à 12h, 8 à 10h, 12h à 3 jours, 12h à 2 jours, 12h à 1 jour, 12 à 18h, 12 à 14h, 1 à 3 jours, ou 1 à 2 jours.

5. Procédé selon l'une quelconque des revendications 1 à 4, dans lequel l'agent inhibiteur de tumeur est un acide nucléique, une protéine ou un peptide.

6. Procédé selon l'une quelconque des revendications 1 à 4, dans lequel l'agent inhibiteur de tumeur est un virus, éventuellement
dans lequel le virus est un virus oncolytique ou un virus trophique tumoral,
éventuellement dans lequel le virus oncolytique est le virus de la vaccine.

7. Procédé selon l'une quelconque des revendications 1 à 6, dans lequel le vecteur exprime en outre le TNF-alpha, un ligand induisant l'apoptose lié au TNF (TRAIL), un gène suicide, la thymidylate synthase, la protéine morphogénique osseuse 4 (BMP4), la thymidine kinase du HSV, un adénovirus oncolytique, l'interleukine-2 (IL-2), l'interleukine-18 (IL-18), l'interleukine-23 (IL-23), l'interféron alpha, l'interféron bêta, l'anticorps bispécifique, l'obestatine, XIAP, la survivine, BCL -2, BCL-XL, GATA-4, IGF-1, EGF, l'hème-oxygénase-1, NF- kB, akt, pi3-k, epha-2, ou une combinaison de ceux-ci.

8. Procédé selon l'une quelconque des revendications 1 à 7, dans lequel les fibroblastes expriment une ou plusieurs cytokines recombinantes, éventuellement
dans lequel la cytokine recombinante est choisie parmi l'interleukine (IL), le facteur de stimulation des colonies (CSF), l'interféron (IFN), le facteur de cellules souches (SCF), les facteurs de croissance tumorale (TGF), le facteur de nécrose tumorale (TNF), ou une combinaison de ceux-ci.

9. Procédé selon l'une quelconque des revendications 1 à 8, dans lequel les fibroblastes de l'étape (a), de l'étape (b) ou des deux sont exposés ou ont été exposés à :
(a) un ou plusieurs inhibiteurs d'histone désacétylase ; ou
(b) une ou plusieurs molécules immunothérapeutiques, éventuellement la molécule immunothérapeutique étant un anticorps, éventuellement l'anticorps étant un anticorps bispécifique.

10. Fibroblastes préparés par le procédé selon l'une quelconque des revendications 1 à 9 pour utilisation dans un procédé de traitement du cancer, dans lequel le procédé comprend l'administration d'une quantité efficace des fibroblastes modifiés à un individu.

11. Fibroblastes à utiliser selon la revendication 10, dans lesquels :
(a) la personne est atteinte d'un cancer ou risque de l'être; et/ou
(b) l'individu reçoit un ou plusieurs agents anticancéreux supplémentaires, l'un ou plusieurs agents anticancéreux supplémentaires comprenant éventuellement une intervention chirurgicale, une chimiothérapie, une radiothérapie, une hormonothérapie et/ou une immunothérapie.
